# EUROPEAN PATENT APPLICATION

(11) **EP 4 233 683 A1**
(43) Date of publication of application: **30.08.2023**
(21) Application number: 21882865.5
(22) Date of filing: 20.10.2021
(51) Int. Cl.: A61B 1/045, A61B 1/24, G06T 7/00, A61C 19/04, A61C 19/06, G16H 30/20, G16H 50/20

(54) **ORAL CONDITION EVALUATION SYSTEM, ORAL CARE RECOMMENDATION SYSTEM, AND ORAL CONDITION NOTIFICATION SYSTEM**

(30) Priority: 22.10.2020 JP 2020177190; 22.10.2020 JP 2020177191; 22.10.2020 JP 2020177192; 22.10.2020 JP 2020177193
(71) Applicant: Sunstar Inc., Takatsuki-shi Osaka 569-1195 (JP)
(72) Inventor: OTA Anri, Takatsuki-shi, Osaka 569-1195 (JP); SHIMIZU Sato, Takatsuki-shi, Osaka 569-1195 (JP); NISHIURA Masahiro, Takatsuki-shi, Osaka 569-1195 (JP); TOSHO Michiaki, Takatsuki-shi, Osaka 569-1195 (JP)
(74) Representative: Murgitroyd & Company
(86) International application number: PCT/JP2021/038774
(87) International publication number: WO 2022/085724

(57) **Abstract**

An oral state evaluation system includes an information acquisition unit and an information analysis unit. The information acquisition unit acquires an oral cavity image including at least a first specific site in the oral cavity from the interface unit as input information. The information analysis unit analyzes the state of the first specific site based on the input information. The information analysis unit estimates the estimation information regarding the state of the second specific site in the oral cavity different from the first specific site from the analysis information of the state of the first specific site.

## Description

### TECHNICAL FIELD

The present disclosure relates to an oral state evaluation system, an oral care recommendation system, and an oral state notification system for evaluating an oral state of a user.

### BACKGROUND ART

There is a known system that analyzes an oral state from an image including the entire oral cavity and assists in determination by an expert such as a dentist. Patent Literature 1 discloses a dental analysis system that extracts a lesion site from an X-ray image of the entire oral cavity of a user captured.

A system for recommending different items according to characteristics of a user is known. Patent Literature 2 discloses an item recommendation system that recommends each user for an item that expectedly increases a satisfaction level of the user based on a plurality of parameters such as user preference.

### CITATIONS LIST

### Patent Literature

Patent Literature 1: Japanese Laid-Open Patent Publication No. 2019 -208831
Patent Literature 2: Japanese Patent No. 6457358

### SUMMARY OF INVENTION

### Technical Problem

It is difficult to obtain information on the state of the entire oral cavity without depending on an expert and an examination instrument such as a probe or an examination device. There is a need for a simple means that allows a user to acknowledge his/her own oral state.

The state of the oral cavity differs between users. Furthermore, even for the same user, the state of the oral cavity changes depending on factors such as time, physical condition, meal contents, and a state before or after implementation of oral care. However, it is not easy to recognize the state of the oral cavity and select an oral care that is appropriate for the current state of the oral cavity without depending on the evaluation of an expert such as a dentist. Selection of an inappropriate oral care for the user's state of the oral cavity may result in effects of the oral care that are lower than expected.

When the oral cavity has a lesion site and the lesion site undergoes an appropriate care, it is expected to remedy the lesion or avoid deterioration in the lesion. However, without an appropriate care, there is a risk that the lesion may deteriorate and a new lesion may be formed in the oral cavity in the future. Therefore, there is a need for encouragement to implement an oral caring action. In an example, outputting of a state of a user after a predetermined time has elapsed encourages the user to implement an oral caring action.

### Solution to Problem

An oral state evaluation system according to the present disclosure includes an information acquisition unit configured to acquire an oral cavity image including at least a first specific site in an oral cavity as input information from an interface unit; and an information analysis unit configured to analyze a state of the first specific site based on the input information, where the information analysis unit estimates estimation information regarding a state of a second specific site in the oral cavity different from the first specific site from analysis information of the state of the first specific site.

An oral care recommendation system according to the present disclosure includes an information acquisition unit configured to acquire input information from an interface unit; an information analysis unit configured to analyze an oral state of a user based on the input information; and an information output unit configured to output an analysis result obtained from the information analysis unit as output information, where the output information includes recommendation information regarding oral care of the user.

An oral state evaluation system according to the present disclosure includes an information acquisition unit configured to acquire an oral cavity image including at least a first specific site in an oral cavity as input information from an interface unit; and an information analysis unit configured to analyze the input information by a learning model that has learned the oral cavity image in advance to evaluate the state in the oral cavity, where the information analysis unit estimates estimation information regarding a state at a second specific site in the oral cavity different from the first specific site.

An oral state notification system according to the present disclosure includes an information acquisition unit configured to acquire input information from an interface unit; an information analysis unit configured to analyze an oral state of a user based on the input information; and an information output unit configured to output an analysis result obtained from the information analysis unit as output information, where the output information includes future information corresponding to a state of the user after a predetermined time has elapsed. Advantageous Effects of Invention

The oral state evaluation system according to the present disclosure provides an oral state evaluation system that evaluates an oral state with a simple means.

With the oral state recommendation system according to the present disclosure, recommendation information regarding oral care is easily obtained in accordance with a user.

The oral state notification system according to the present disclosure encourages to implement an oral caring action.

### BRIEF DESCRIPTION OF DRAWINGS

Fig. 1 is a block diagram showing configurations of an oral state evaluation system according to a first embodiment, an oral state recommendation system according to a second embodiment, an oral state evaluation system according to a third embodiment, and an oral state notification system according to a fourth embodiment.
Fig. 2 is a view illustrating an oral cavity image as an example of input information.
Fig. 3 is a partial enlarged schematic view of the oral cavity image of Fig. 2.
Fig. 4 is a view showing an example of an oral state in a case where there is teeth crowding.
Fig. 5 is a view showing an example of an oral state in a case where there is no teeth crowding.
Fig. 6 is a view showing an example of an oral state in a case where there is interdental space.
Fig. 7 is a view showing an example of an oral state in a case where there is no interdental space.
Fig. 8 is a view showing an example of an oral state in a case where there is gingival recession.
Fig. 9 is a view showing an example of an oral state in a case where there is no gingival recession.
Fig. 10 is a flowchart illustrating an example of an oral care recommendation process executed by an information analysis unit.
Fig. 11 is a schematic view illustrating an example of presentation on a display unit.

### DESCRIPTION OF EMBODIMENTS

### Example of Applicable Form of Oral State Evaluation System in First Embodiment

(1-1) An oral state evaluation system according to the present disclosure includes an information acquisition unit configured to acquire an oral cavity image including at least a first specific site in an oral cavity as input information from an interface unit; and an information analysis unit configured to analyze a state of the first specific site based on the input information; where the information analysis unit estimates estimation information regarding a state of a second specific site in the oral cavity different from the first specific site from analysis information of the state of the first specific site.

In the oral state evaluation system described above, the information analysis unit estimates the estimation information regarding the state of the second specific site in the oral cavity different from the first specific site from the oral cavity image including the first specific site. The oral state of the entire oral cavity can be evaluated regardless of whether or not the oral cavity image includes the second specific site. Therefore, the oral state can be evaluated by a simple means.

(1-2) According to one example of the oral state evaluation system, the oral cavity image includes a tooth image and a gum image in the oral cavity.

According to the oral state evaluation system described above, since the oral cavity image includes an image of an appropriate site in the oral cavity, the oral state can be appropriately evaluated.

(1-3) According to one example of the oral state evaluation system, the tooth image includes a central incisor image, a lateral incisor image, and a canine image in the oral cavity.

According to the oral state evaluation system described above, since the oral cavity image includes an image of appropriate teeth in the oral cavity, the oral state can be appropriately evaluated.

(1-4) According to one example of the oral state evaluation system, the second specific site includes at least one of a premolar and a molar in the oral cavity, and the estimation information includes at least one of information regarding the premolar and information regarding the molar in the oral cavity.

According to the oral state evaluation system described above, since at least one of information regarding the premolar and information regarding the molar in the oral cavity is estimated, the oral state can be appropriately evaluated.

(1-5) According to one example of the oral state evaluation system, the estimation information includes at least one of information regarding presence or absence or a degree of interdental space, gingival recession, teeth crowding, gingival inflammation, insufficient brushing, bruxism, caries, hyperesthesia, bad breath, and coloring at the second specific site, information regarding whether or not periodontal disease is present, information regarding whether or not chewing function is normal, information regarding whether or not occlusion is normal, information regarding whether or not swallowing function is normal, and information regarding a state of teeth, gums, and oral mucosa corresponding to the second specific site.

According to the oral state evaluation system described above, at least one of the states of the tooth and the gum at the second specific site can be estimated. Therefore, the oral state can be appropriately evaluated.

(1-6) One example of the oral state evaluation system further includes an information storage unit configured to store a learning model in which the oral cavity image is learned in advance to evaluate a state in the oral cavity, where the information analysis unit analyzes the input information with the learning model.

According to the oral state evaluation system described above, the estimation information is estimated by the learning model. Therefore, the oral state can be more appropriately evaluated.

(1-7) According to one example of the oral state evaluation system, the input information further includes at least one of information regarding a lifestyle of a user, information regarding an intraoral endocrine of the user, information regarding an intraoral bacterial flora of the user, information regarding an attribute of the user, and information obtained by a sensor that detects a state in the oral cavity.

According to the oral state evaluation system described above, the oral state can be more appropriately evaluated.

(1-8) One example of the oral state evaluation system further includes an information output unit configured to output information corresponding to the analysis information and the estimation information as output information, where the information output unit outputs the output information to at least the interface unit.

According to the oral state evaluation system described above, the user can easily recognize the output information.

(1-9) According to one example of the oral state evaluation system, the output information includes at least one of information regarding a current oral state of a user, information regarding prediction of a future oral state of the user, information regarding an oral care method for the user, and information regarding a health state of the user affected by the oral state of the user.

According to the oral state evaluation system described above, the user can appropriately recognize the output information related to the oral state.

### First Embodiment

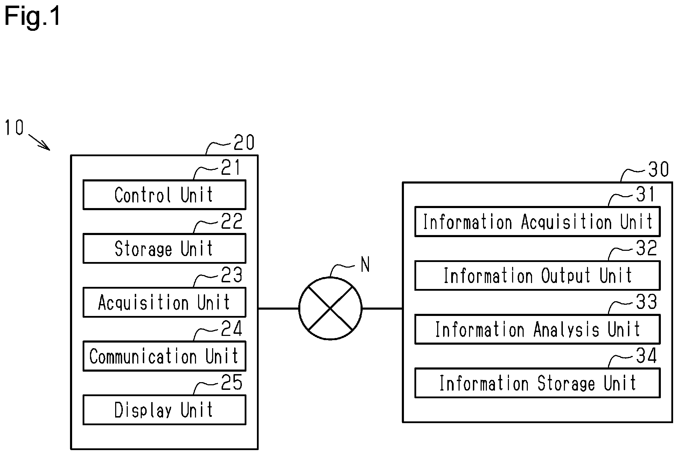

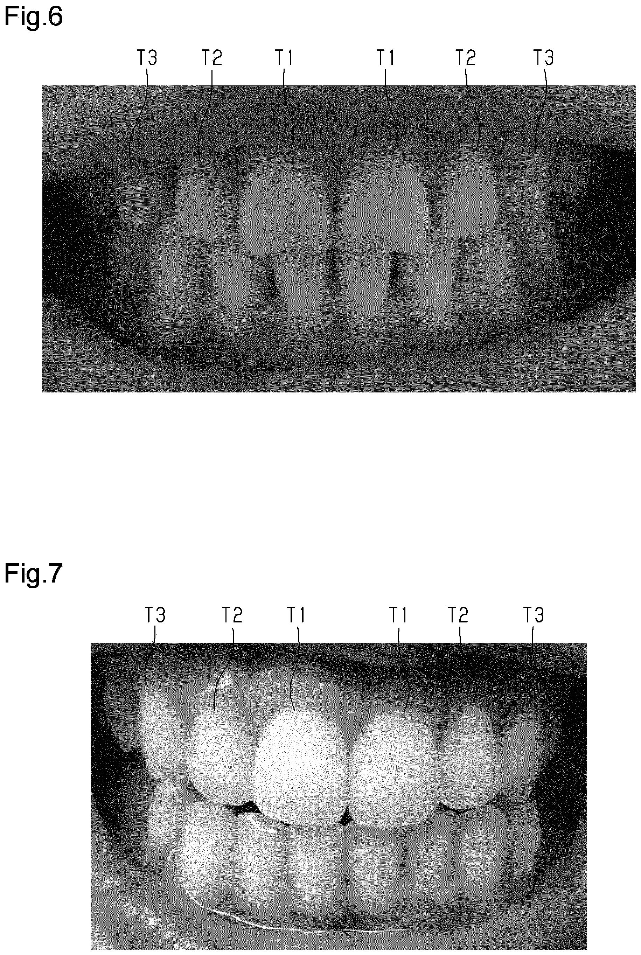

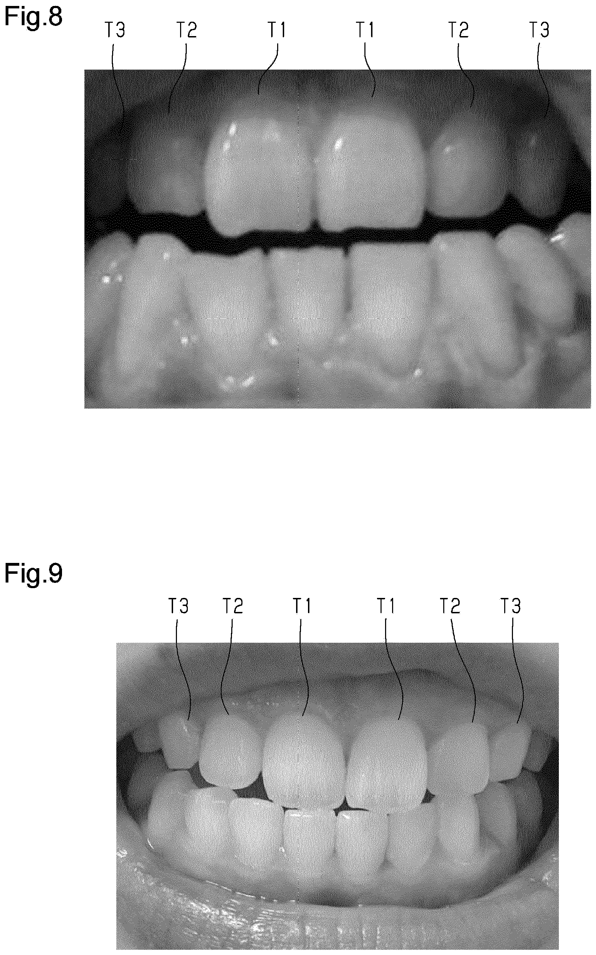

An oral state evaluation system 10 according to a first embodiment will be described with reference to Figs. 1 to 9. The oral state evaluation system 10 is a system that analyzes input information I and estimates estimation information E based on analysis information A obtained from the input information I. The oral state evaluation system 10 may calculate output information O corresponding to the analysis information A and the estimation information E, and output the output information O to a predetermined configuration. A main element forming the oral state evaluation system 10 is a server 30. Preferably, the oral state evaluation system 10 includes an interface unit 20 for exchanging information with the server 30. In one example, the interface unit 20 is a smart device configured to be carried by the user. The smart device includes a tablet terminal or a smartphone. In another example, the interface unit 20 is a personal computer. The personal computer is installed at the user's residence, a store, or a dental office. The store includes a store that sells oral care items or a store that sells other items. The interface unit 20 and the server 30 are configured to be able to communicate with each other using, for example, the Internet connection N. In another example, the interface unit 20 and the server 30 are integrally configured.

The interface unit 20 includes a control unit 21, a storage unit 22, an acquisition unit 23, a communication unit 24, and a display unit 25. The control unit 21 includes an arithmetic processing unit that executes a predetermined control program. The arithmetic processing unit includes, for example, a CPU, a GPU, or an MPU.

The storage unit 22 stores various control programs executed by the control unit 21 and information used for various control processes. The storage unit 22 includes, for example, a nonvolatile memory and a volatile memory. The nonvolatile memory includes, for example, at least one of a ROM, an EPROM, an EEPROM, and a flash memory. The volatile memory includes, for example, a RAM.

The acquisition unit 23 acquires the input information I from the user. The acquisition unit 23 has any configuration for acquiring the input information I of the user. In the first example, it is a camera capable of acquiring an oral cavity image P of the user's oral cavity. The oral cavity image P includes a still image and a moving image. The oral cavity image P includes a three-dimensional image having depth information or a panoramic image formed by joining a plurality of images. The camera may be a camera mounted on the smart device, or may be a separate independent camera. The independent camera is, for example, a pen type camera in which a camera is provided at a distal end portion suitable for photographing the oral cavity or a camera capable of photographing a range of 360°. The independent camera is configured to be capable of wired or wireless communication with the control unit 21. The oral cavity image P captured by the independent camera is transmitted to the control unit 21 through wired or wireless communication. In the second example, the acquisition unit 23 is a user interface configured to allow the user to input or select a character or the like. The input information I of the user may further include at least one of information regarding the lifestyle of the user, information regarding the intraoral endocrine of the user, information regarding the intraoral bacterial flora of the user, answer information to a predetermined question, and information regarding an attribute of the user. The information regarding the lifestyle of the user includes information regarding the user's meal content, oral caring action, and wake-up or sleeping time. The information regarding the intraoral endocrine of the user includes information on the amount of saliva, viscosity, hydrogen ion concentration, amount of antimicrobial component, and amount of component related to tooth remineralization. The information regarding the intraoral bacterial flora of the user includes information regarding the amount and type of main bacteria present in saliva, dental plaque, or mucosa, and information regarding the type and amount of antibodies of the main bacteria. The information regarding the intraoral endocrine of the user and the information regarding the intraoral bacterial flora of the user may be a result of an interview with the user, or may be result information obtained by examining the intraoral endocrine or the like existing in the oral cavity of the user by a predetermined means. The answer information regarding the predetermined question includes a question about the state of the user's gums, a preferred brushing method during oral care, the number of times teeth are brushed in a day, the time taken for brushing teeth each time, the time at which teeth are brushed, the frequency of use of oral care items, and the presence or absence of dentures. The question regarding the state of the user's gums includes, for example, the presence or absence of bleeding from the gums at the time of brushing or eating or drinking. The information regarding the attribute of the user is, for example, the user's own age, sex, height, weight, dominant arm, and medical history. In the third example, the acquisition unit 23 is a sensor that detects a state in the oral cavity. The sensor is, for example, a fluorescent sensor and a temperature sensor. The fluorescence sensor emits light of a predetermined wavelength, quantifies the amount of light, and measures the distribution and amount of a predetermined object in the oral cavity. The predetermined object is, for example, stain or plaque. The temperature sensor measures a temperature in the oral cavity. The sensor is configured to be capable of wired or wireless communication with the control unit 21. Information obtained by the sensor is transmitted to the control unit 21 through wired or wireless communication. In the present embodiment, the acquisition unit 23 may be configured by combining two or more of the first to third examples. The sensor may be further configured to be able to measure at least one of chewing force, biting force, amount of bleeding from gums, bad breath, strength of brush pressure at the time of brushing, or movement of toothbrush at the time of brushing.

The communication unit 24 is configured to be able to communicate with the outside of the interface unit 20 based on the control of the control unit 21. The communication unit 24 is configured to be able to communicate via the Internet connection N. The communication unit 24 may be configured to be able to communicate with the server 30 of the interface unit 20 through wired communication or wireless communication. The communication unit 24 transmits, for example, the input information I of the user acquired by the acquisition unit 23 based on the control of the control unit 21, and receives the output information O from the server 30.

The display unit 25 displays various types of information based on the control of the control unit 21. The various types of information are, for example, information regarding the input information I of the user and information regarding the output information O from the server 30. In one example, the display unit 25 includes a display. The display of the display unit 25 may include a touch panel. In a case where a part of the display unit 25 is formed of a touch panel, that part may also function as a user interface of the acquisition unit 23.

The user communicates with the server 30 by, for example, inputting a predetermined URL to the interface unit 20 or reading a QR code (registered trademark) by the interface unit 20. The user may start communication with the server 30 by selecting an icon displayed on the display unit 25.

The server 30 includes an information acquisition unit 31, an information output unit 32, an information analysis unit 33, and an information storage unit 34. The information acquisition unit 31 is configured to be able to acquire information. In one example, the information acquisition unit 31 acquires information from the communication unit 24 of the interface unit 20. The information output unit 32 is configured to be able to output information. In one example, the information output unit 32 outputs information to the communication unit 24 of the interface unit 20.

The information analysis unit 33 executes various analyses and controls. The information analysis unit 33 includes an arithmetic processing unit that executes a predetermined control program. The arithmetic processing unit includes, for example, a CPU or an MPU. The information analysis unit 33 is configured to be able to analyze the input information I from the user. In the first example, the information analysis unit 33 analyzes the input information I using the learning model M by machine learning. The input information I in this case includes the oral cavity image P of the user. In one example, the learning model M is a supervised learning model, which is one of the models of machine learning. In the second example, the information analysis unit 33 analyzes the input information I with reference to a correspondence table stored in the information storage unit 34. The input information I in this case includes, instead of or in addition to the oral cavity image P, at least one of information regarding the lifestyle of the user, information regarding the user's intraoral endocrine, information regarding the user's intraoral bacterial flora, and answer information to a predetermined question. The correspondence table is a table in which the input information I and the analysis information A are associated with each other. In the correspondence table, the input information I and the analysis information A may be associated with the estimation information E. In a case where the input information I is further included in addition to the oral cavity image P, in one example, the learning model M further includes another learning model that analyzes the input information I other than the oral cavity image P. Another learning model is configured to be able to output, for example, a parameter for correcting the analysis information A of the oral cavity image P. In another example, the learning model M is configured as a model capable of executing multimodal learning for performing learning by combining both the oral cavity image P and the input information I not including the oral cavity image P.

The information storage unit 34 stores at least one of the learning model M, the correspondence table, and various types of information. The information analysis unit 33 refers to the learning model M, the correspondence table, and various types of information stored in the information storage unit 34 as necessary.

The analysis of the input information I including the oral cavity image P using the learning model M executed by the information analysis unit 33 will be described. The step of analysis executed by the information analysis unit 33 includes a number of processes. The processes include a first process of detecting an oral region R from the oral cavity image P of the user, a second process of calculating the analysis information A by analyzing the oral region R included in the oral cavity image P, a third process of estimating the estimation information E from the analysis information A, and a fourth process of calculating the output information O corresponding to the analysis information A and the estimation information E.

The first process is executed by the information analysis unit 33 with any means. In one example, the oral region R is detected from the oral cavity image P of the oral cavity of the user using a learned model suitable for detecting the face. The oral region R includes a first oral region R1 including the teeth of the user's upper jaw and a second oral region R2 including the teeth of the user's lower jaw. The learned model for detecting the face is introduced into the information analysis unit 33 by, for example, an application programming interface (API).

In the first process, in a case in which the oral region R cannot be acquired from the oral cavity image P, the information analysis unit 33 outputs a content indicating the result to the display unit 25 of the interface unit 20 via the information output unit 32. In one example, when the oral region R cannot be recognized because the oral region R is not included in the oral cavity image P, the oral cavity image P is blurry, or the brightness of the oral cavity image P is not appropriate, the display unit 25 of the interface unit 20 outputs information requesting the user to again input the input information I including the oral cavity image P . In a case where one of the first oral region R1 and the second oral region R2 can be acquired and the other cannot be acquired, the information analysis unit 33 may determine that the first process has been completed, or may output, to the interface unit 20, information requesting the user to again input the input information I.

In the second process, the information analysis unit 33 analyzes the oral cavity image P. The information analysis unit 33 analyzes the oral cavity image P using the learning model M stored in the information storage unit 34. The analysis information A, which is the analysis result, includes at least one of information regarding presence or absence or a degree of interdental space, gingival recession, teeth crowding, gingival inflammation, insufficient brushing, bruxism, caries, hyperesthesia, bad breath, and coloring at the first specific site in the oral region R, and information regarding a state of teeth, gums, and oral mucosa corresponding to the first specific site. The information regarding the state of the teeth, gums, and oral mucosa includes at least one of, for example, the presence or absence or degree of tooth wear, tooth loss, and tooth fracture, color, dryness, and texture. The texture includes, for example, different textures including a hard texture that is felt to be firm when touched with a finger and a soft texture that is felt to be tender when touched with a finger. In a case where the results differ between the analysis information A in the first oral region R1 and the analysis information A in the second oral region R2, one of the two types of the analysis information A may be set in advance to be used. Alternatively, the two types of analysis information A may be quantified and an average thereof may be used. The two types of analysis information A may be quantified and the analysis information A having a larger absolute value may be used.

In the third process, the information analysis unit 33 estimates the estimation information E from the input information I and the analysis information A. The estimation information E includes information on at least one of the presence or absence, the degree, and the probability of a predetermined state. The estimation information E includes at least one of information regarding presence or absence or a degree of interdental space, gingival recession, teeth crowding, gingival inflammation, insufficient brushing, bruxism, caries, hyperesthesia, bad breath, and coloring at the second specific site different from the first specific site, or information regarding a state of teeth, gums, and oral mucosa corresponding to the second specific site. The second specific site includes at least one of a premolar and a molar in the oral cavity. The estimation information E includes at least one of information on a premolar and information regarding a molar in the oral cavity. The estimation information E may include at least one of information regarding whether or not periodontal disease is present in the oral cavity of the user, information regarding whether or not chewing function is normal, information regarding whether or not occlusion is normal, and information regarding whether or not swallowing function is normal, The information on whether or not periodontal disease is present is information on at least one of the size, depth, and number of so-called periodontal pockets that are formed between the gingiva and the tooth. The information on whether or not the chewing function is normal is, for example, information on chewing force, presence or absence of unbalanced chewing, and chewing sound. The information on whether or not occlusion is normal is, for example, information on the biting force and the fitting of the tooth of the upper jaw with the tooth of the lower jaw. The information on whether or not the swallowing function is normal is, for example, information on presence or absence of swallowing difficulty.

In the fourth process, the information analysis unit 33 calculates the output information O corresponding to at least one of the analysis information A and the estimation information E. The output information O includes at least one of information regarding a current oral state of a user, information regarding prediction of a future oral state of the user, information regarding an oral care method for the user, and information regarding a health state of the user affected by the oral state of the user.

The learning model M for analyzing the oral region R will be described. The learning model M includes a plurality of learned models. The learning model M includes a first learned model M1 that determines whether or not the oral region R can be evaluated and a second learned model M2 that actually evaluates the oral region R.

The first learned model M1 includes at least one of a learning model M11 that determines whether or not the oral region R of the oral cavity image P has an analyzable image quality, a learning model M12 that determines whether or not the teeth crowding evaluation is possible, a learning model M13 that determines whether or not the interdental space evaluation of the teeth of the upper jaw is possible, a learning model M14 that determines whether or not the interdental space evaluation of the teeth of the lower jaw is possible, and a learning model M15 that determines whether or not the gingival recession evaluation is possible.

The second learned model M2 includes at least one of a learning model M21 that determines presence or absence of teeth crowding in the tooth of the upper jaw, a learning model M22 that determines presence or absence of teeth crowding in the tooth of the lower jaw, a learning model M23 that determines presence or absence of an interdental space in the tooth of the upper jaw, a learning model M24 that determines presence or absence of an interdental space in the tooth of the lower jaw, and a learning model M25 that determines presence or absence of interdental recession.

The creation of the learning model M will be described. The learning model M was created based on approximately 10,000 oral cavity images P. The developer classified the oral cavity images P into a learning image, a verification image, and a test image. The learning image is supervised data at the time of creating the learning model M. The verification image is an image for correcting the operation of the learning model M based on the learning image. The test image is an image for finally confirming the operation of the learning model M. The test image is used, for example, to determine whether or not the learning model M is causing overfitting. The learning image accounts for about 56% of the whole. The verification image accounts for about 24% of the whole. The test image accounts for 20% of the whole. In the present embodiment, the learning model M outputs a result of class classification. The learning model M outputs at least one of information regarding presence or absence or a degree of interdental space, gingival recession, teeth crowding, gingival inflammation, insufficient brushing, bruxism, caries, hyperesthesia, bad breath, and coloring, information regarding a state of teeth, gums, and oral mucosa corresponding to the second specific site, information regarding whether or not periodontal disease is present in the oral cavity of the user, information regarding whether or not chewing function is normal, information regarding whether or not occlusion is normal, and information regarding whether or not swallowing function is normal.

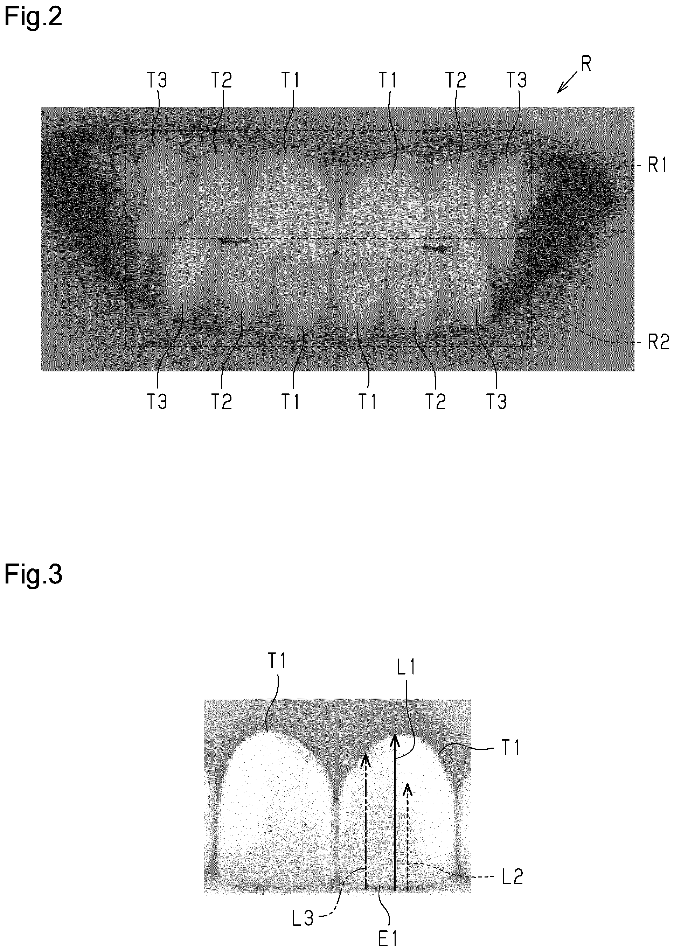

Fig. 2 illustrates an example of the oral cavity image P of the user. The oral cavity image P includes at least a first specific site in the oral cavity. The first specific site includes the central incisor T1, the gum corresponding to the central incisor T1, the lateral incisor T2, the gum corresponding to the lateral incisor T2, the canine T3, and the gum corresponding to the canine T3. The oral cavity image P includes a tooth image and a gum image in the oral cavity. The tooth image includes an image of the central incisor T1, an image of the lateral incisor T2, and an image of the canine T3 in the oral cavity. The gum image includes images of the gums corresponding to the central incisor T1, the lateral incisor T2, and the canine T3. The oral cavity image P includes at least one of the four first specific sites located at upper or lower and left or right positions in the oral cavity of the user.

A preferable range of the tooth image included in the oral cavity image P will be described with reference to Fig. 3. In the determination of the presence or absence of the teeth crowding, it is preferred that the oral cavity image P includes a range from the end portion E1 of the central incisor T1 to the broken line L2. In the determination of the presence or absence of the interdental space, it is preferred that the oral cavity image P includes a range from the end portion E1 of the central incisor T1 to the double-dashed line L3. In the determination of the presence or absence of gingival recession, it is preferred that the oral cavity image P includes a range from the end portion E1 of the central incisor T1 to the solid line L1. When the oral cavity image P includes the image of the central incisor T1 in each range described above, the oral cavity image P includes the image of the corresponding gum.

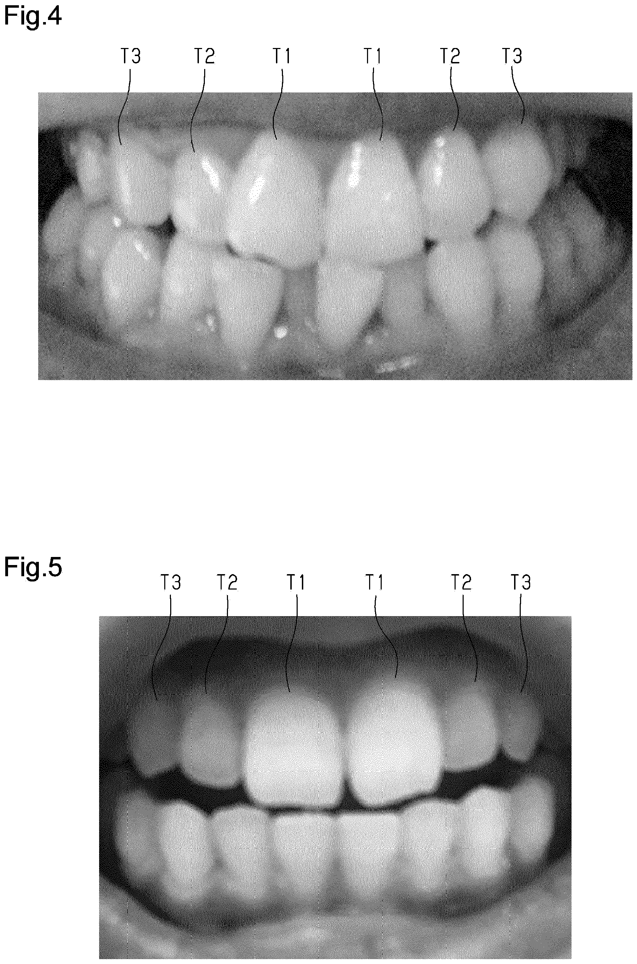

Each oral cavity image P was supervised by an expert who analyzes oral cavities. Figs. 4 to 9 each illustrate an example of a supervised oral cavity image P. An expert who analyzes oral cavities is, for example, a dentist, a dental hygienist, a researcher who studies oral states, or a developer who develops oral care products. The expert who analyzes oral cavities performed a determination of the degree of teeth crowding, a determination of the degree of gingival recession, and a determination of the degree of interdental space for each of the first oral region R1 and the second oral region R2 in the oral cavity image P of the oral cavity. In the determination of the degree of teeth crowding, the expert who analyzes oral cavities performed a determination based on the type of teeth crowding. In the determination of gingival recession, the expert who analyzes oral cavities performed a determination of a plurality of stages from no gingival recession to complete gingival recession. The expert who analyzes oral cavities performed an evaluation of a plurality of stages from no interdental space to severe interdental space with respect to each of the upper jaw and the lower jaw.

The state of the oral cavity at the first specific site is correlated with the state of the oral cavity at the second specific site different from the first specific site. In one example, a test conducted on seventy-five women in their 40's to 70's shows that the state of the gingiva in the front tooth and the state of the gingiva in the back tooth are highly correlated. That is, when the gingival recession occurs at the first specific site, the gingival recession also occurs at the second specific site. The same applies to the degree of interdental space and teeth crowding.

The information analysis unit 33 outputs the analysis information A and the estimation information E to a predetermined configuration. In the first example, the information analysis unit 33 outputs the estimation information E to the information output unit 32. In the second example, the information analysis unit 33 outputs the estimation information E to the information storage unit 34. In the third example, the information analysis unit 33 outputs the estimation information E to both the information output unit 32 and the information storage unit 34.

The analysis information A and the estimation information E are associated with the output information O. The output information O will be described. The output information O includes at least one of information regarding a current oral state of the user, information regarding prediction of a future oral state of the user, information regarding an oral care method for the user, and information regarding a health state of the user affected by the oral state of the user. The information regarding the current oral state of the user includes information regarding at least one of presence or absence of teeth crowding, presence or absence of gingival recession, and presence or absence of an interdental space in the entire oral cavity of the user. The information regarding the current oral state of the user may further include at least one of presence or absence of gingival inflammation, presence or absence of insufficient brushing, state of insufficient brushing, presence or absence of bruxism, presence or absence of hyperesthesia, and presence or absence of bad breath.

The information regarding prediction of the future oral cavity of the user includes an estimated image showing an oral state of the user after a predetermined period has elapsed. The information regarding the oral care method for the user includes information regarding an oral care product and a usage method suitable for the oral state of the user. The information regarding the health state of the user affected by the oral state of the user includes, for example, information regarding a health state other than the oral cavity related to the periodontal disease or the oral state.

The correspondence between the analysis information A and the estimation information E, and the output information O is executed by the information analysis unit 33 with any means. In the first example, it is executed using a correspondence table in which the analysis information A and the estimation information E are associated with the output information O in advance by a researcher who studies oral states or a developer who develops oral care products. In the second example, the correspondence between the analysis information A and the estimation information E, and the output information O is executed using a model of machine learning.

The operation of the oral state evaluation system 10 of the present embodiment will be described. The user inputs the input information I to the acquisition unit 23. The control unit 21 controls the communication unit 24 to output the input information I to the server 30. The server 30 acquires the input information I with the information acquisition unit 31. The information analysis unit 33 analyzes the input information I to calculate the analysis information A of the first specific site using the learning model M stored in the information storage unit 34. The information analysis unit 33 calculates the estimation information E including the second specific site from the analysis information A. The information analysis unit 33 calculates the output information O corresponding to the analysis information A and the estimation information E. The server 30 outputs the output information O from the information output unit 32 to the interface unit 20. The control unit 21 acquires the output information O from the communication unit 24 and displays the output information O on the display unit 25. This allows the user to recognize the output information O via the display of the display unit 25.

### Modification of First Embodiment

The description related to the first embodiment exemplifies applicable forms of an oral state evaluation system according to the present disclosure and is not intended to limit the forms. The present disclosure may take, other than the embodiment, a form in which, for example, modifications of the embodiment shown below and at least two modifications that do not contradict each other are combined.

The learning model M may be configured to output a result of regression analysis. In this case, the learning model M quantifies and outputs at least one of the degree of teeth crowding, the degree of interdental space, and the degree of gingival recession.

At least one of the learned model M1 and the learned model M2 may be a model learned by unsupervised learning or reinforcement learning. At least one of the learning models M11 to M15 and the learning models M21 to M25 may be a model learned by unsupervised learning or reinforcement learning.

The learning model M may include a first learned model M1 that determines whether or not evaluation is possible and a second learned model M2 that performs evaluation without distinguishing the first oral region R1 and the second oral region R2 in the oral cavity image P. The learning model M may include a first learned model M1 that recognizes a set of a tooth and gum in the oral cavity image P and determines whether or not evaluation is possible and a second learned model M2 that performs evaluation. In addition, the learning model M may include a learning model M that recognizes each set of a tooth and gum in the oral region R and executes analysis when a majority of the teeth and gums can be evaluated. In addition, the analysis information A of each set of a tooth and gum may be set in advance to be used. The analysis information A of each set of a tooth and gum may be quantified and an average thereof may be used, or the analysis information A of each set of a tooth and gum may be quantified and the analysis information A having a large absolute value may be used.

The learning model M may be configured to display the oral cavity image P as the output information O in a pseudo color. In one example, a region used for analysis using the learning model M in the oral cavity image P or a region including interdental space, gingival recession, or teeth crowding are displayed in red. The user can easily recognize the region used for the analysis and the region having a problem with the oral state.

In a case where the output information O includes information regarding the user's oral care product and usage method, it may be configured to include purchase information for purchasing the corresponding oral care product. In one example, the purchase information is information on a store where the corresponding oral care product can be purchased or tried. In another example, the purchase information is site information on a web that sells the corresponding oral care product.

The input information I may be acquired via an (Internet of things) loT device. In one example, the input information I is acquired by connecting an loT device to an oral care item used for brushing. In one example, the input information I includes information on the number of times teeth are brushed in a day, and the frequency of use of the oral care item. The loT device may transmit the input information I to the acquisition unit 23, or may transmit the input information I to the server 30.

At least one of the learning model M, the correspondence table, and various types of information may be stored in a place other than the information storage unit 34 of the server 30. In one example, it is stored in a storage unit provided in the interface unit 20. In another example, it is stored in a database configured in an external environment.

The input information I may include first input information 11 including user's current state information and second input information I2 being user's past state information. In a case where the first input information 11 and the second input information I2 include the oral cavity image P of the user, the oral cavity image P included in the second input information I2 is an image of the oral cavity of the user taken at a predetermined time before the first input information 11 is acquired. In a case where the first input information 11 and the second input information I2 include information regarding oral care of the user, the information regarding oral care included in the second input information I2 is information regarding oral care a predetermined time before the first input information 11 is acquired. The predetermined time is, for example, a time of one month or more. In another example, the predetermined time is a time of one year or more. The second input information I2 may be transmitted to the server 30 via the communication unit 24 before the acquisition unit 23 acquires the first input information 11, or may be transmitted to the server 30 via the communication unit 24 simultaneously with the transmission of the first input information 11. In this case, the interface unit 20 further includes a storage unit configured to store at least the second input information I2. The input information I may further include third input information I3 acquired a predetermined time before acquisition of the second input information I2. The information analysis unit 33 calculates the analysis information A and the estimation information E using at least one of the pieces of the first input information 11 to the third input information I3. The accuracy is further improved by adding the third input information I3. The interval of the predetermined time may be changed by each piece of input information I. The input information I may further include input information I including fourth input information and so on.

### Example of Applicable Form of Oral Care Recommendation System in Second Embodiment

(2-1) An oral care recommendation system according to the present disclosure includes an information acquisition unit configured to acquire input information from an interface unit; an information analysis unit configured to analyze an oral state of a user based on the input information; and an information output unit configured to output an analysis result obtained from the information analysis unit as output information, where the output information includes recommendation information regarding oral care of the user.

According to the oral care recommendation system described above, the output information that is obtained by analyzing the oral state of the user and is output includes recommendation information regarding the oral care. Therefore, recommendation information regarding oral care corresponding to the user is easily obtained.

(2-2) According to an example of the oral care recommendation system, the recommendation information includes oral care item recommendation information for recommending an oral care item in accordance with the oral state of the user.

According to the oral care recommendation system described above, since the output information includes the oral care item recommendation information, the user can select a suitable oral care item by a simple means.

(2-3) According to an example of the oral care recommendation system, the oral care item includes at least one of a toothbrush, an interdental cleaning tool, and an oral washing agent.

According to the oral care recommendation system described above, each user can select an oral care item suitable for the user in a further specified manner.

(2-4) According to an example of the oral care recommendation system, the recommendation information further includes information regarding a usage method of the oral care item.

According to the oral care recommendation system described above, the user can easily acquire an appropriate usage method of the oral care item.

(2-5) According to an example of the oral care recommendation system, the input information includes an image including a first specific site in an oral cavity of the user, and the information regarding a usage method of the oral care item includes a usage method of the oral care item at least at the first specific site.

According to the oral care recommendation system described above, a usage method of an appropriate oral care item is acquired from an image including a specific site in the oral cavity.

(2-6) According to an example of the oral care recommendation system, the input information includes information regarding oral care of the user, and the output information further includes answer information corresponding to the information regarding the oral care of the user.

According to the oral care recommendation system described above, since the output information includes answer information corresponding to the information regarding the oral care, the user can implement a more suitable oral care.

(2-7) According to an example of the oral care recommendation system, the information regarding the oral care of the user includes at least one of information regarding an oral care item using action of the user, information regarding a lifestyle of the user, information regarding intraoral endocrine of the user, information regarding intraoral bacterial flora of the user, information regarding an attribute of the user, and information obtained by a sensor that detects a state in the oral cavity.

According to the oral care recommendation system described above, answer information is set based on information regarding an appropriate oral care.

(2-8) According to an example of the oral care recommendation system, the information output unit outputs the output information to the interface unit, and the interface unit includes a display unit configured to display the output information.

According to the oral care recommendation system described above, the user can easily recognize the output information.

### Second Embodiment

An oral care recommendation system 10 according to a second embodiment will be described with reference to Figs. 1 to 11. The oral care recommendation system 10 is a system that analyzes the acquired input information I and outputs the output information O based on the analysis information A obtained from the input information I. The output information O includes recommendation information RI regarding oral care of the user. The oral care recommendation system 10 may estimate the estimation information E regarding the state in the oral cavity from the analysis information A, and further output the output information O based on the estimation information E. A main element forming the oral care recommendation system 10 is a server 30. Preferably, the oral care recommendation system 10 includes an interface unit 20 for exchanging information with the server 30. In one example, the interface unit 20 is a smart device configured to be carried by the user. The smart device includes a tablet terminal or a smartphone. In another example, the interface unit 20 is a personal computer. The personal computer is installed at the user's residence, store, or dental office. A store includes a store that sells oral care items or a store that sells other items. The interface unit 20 and the server 30 are configured to be able to communicate with each other using, for example, the Internet connection N. In another example, the interface unit 20 and the server 30 are integrally configured.

The interface unit 20 includes a control unit 21, a storage unit 22, an acquisition unit 23, a communication unit 24, and a display unit 25. The control unit 21 includes an arithmetic processing unit that executes a predetermined control program. The arithmetic processing unit includes, for example, a CPU, a GPU, or an MPU.

The storage unit 22 stores various control programs executed by the control unit 21 and information used for various control processes. The storage unit 22 includes, for example, a nonvolatile memory and a volatile memory. The nonvolatile memory includes, for example, at least one of a ROM, an EPROM, an EEPROM, and a flash memory. The volatile memory includes, for example, a RAM.

The acquisition unit 23 acquires the input information I from the user. The acquisition unit 23 has any configuration for acquiring the input information I of the user. In the first example, it is a camera capable of acquiring an oral cavity image P of the user's oral cavity. The oral cavity image P includes a still image and a moving image. The oral cavity image P includes a three-dimensional image having depth information or a panoramic image formed by joining a plurality of images. The camera may be a camera mounted on the smart device, or may be a separate independent camera. The independent camera is, for example, a pen type camera in which a camera is provided at a distal end portion suitable for photographing the oral cavity or a camera capable of photographing a range of 360°. The independent camera is configured to be capable of wired or wireless communication with the control unit 21. The oral cavity image P captured by the independent camera is transmitted to the control unit 21 through wired or wireless communication. The acquisition unit 23 of the second example acquires information regarding the oral care of the user. In the second example, the acquisition unit 23 is a user interface configured to allow, for example, the user to input or select a character or the like. The information regarding the oral care of the user includes at least one of information regarding an oral care item using action of the user, information regarding the lifestyle of the user, information regarding the user's intraoral endocrine, information regarding the user's intraoral bacterial flora, information regarding an answer to a predetermined question, and information regarding the attribute of the user. The information regarding an oral care item using action of the user includes at least one of a model number, a frequency of use, the number of times used, and a using time of the user's oral care item. The oral care item includes, for example, at least one of a toothbrush, an interdental cleaning tool, and an oral washing agent. The information regarding the lifestyle of the user includes information regarding the user's meal content and wake-up or sleeping time. The information regarding the intraoral endocrine of the user includes information on the amount of saliva, viscosity, hydrogen ion concentration, amount of antimicrobial component, and amount of component related to tooth remineralization. The information regarding the intraoral bacterial flora of the user includes information regarding the amount and type of main bacteria present in saliva, dental plaque, or mucosa, and information regarding the type and amount of antibodies of the main bacteria. The information regarding the intraoral endocrine of the user and the information regarding the intraoral bacterial flora of the user may be a result of an interview with the user, or may be result information obtained by examining the intraoral endocrine or the like existing in the oral cavity of the user by a predetermined means. The answer information regarding the predetermined question includes a question about the state of the user's gums, a preferred brushing method during oral care, the number of times teeth are brushed in a day, the time taken for brushing teeth each time, the time at which teeth are brushed, the frequency of use of oral care items, and the presence or absence of dentures. The question regarding the state of the user's gums includes, for example, the presence or absence of bleeding from the gums at the time of brushing or eating or drinking. The information regarding the attribute of the user is, for example, the user's own age, sex, height, weight, and medical history. In the third example, the acquisition unit 23 is a sensor that detects a state in the oral cavity. The sensor is, for example, a fluorescent sensor and a temperature sensor. The fluorescence sensor emits light of a predetermined wavelength, quantifies the amount of light, and measures the distribution and amount of a predetermined object in the oral cavity. The predetermined object is, for example, stain or plaque. The temperature sensor measures a temperature in the oral cavity. The sensor is configured to be capable of wired or wireless communication with the control unit 21. Information obtained by the sensor is transmitted to the control unit 21 by wired or wireless communication. In the present embodiment, the acquisition unit 23 may be configured by combining two or more of the first to third examples. The sensor may be further configured to be able to measure at least one of chewing force, biting force, amount of bleeding from gums, bad breath, strength of brush pressure at the time of brushing, or movement of toothbrush at the time of brushing.

The communication unit 24 is configured to be able to communicate with the outside of the interface unit 20 based on the control of the control unit 21. The communication unit 24 is configured to be able to communicate via the Internet connection N. The communication unit 24 may be configured to be able to communicate with the server 30 of the interface unit 20 through wired communication or wireless communication. The communication unit 24 transmits, for example, the input information I of the user acquired by the acquisition unit 23 based on the control of the control unit 21, and receives the output information O from the server 30.

The display unit 25 displays various types of information based on the control of the control unit 21. The various types of information are, for example, information regarding the input information I of the user and information regarding the output information O from the server 30. In one example, the display unit 25 includes a display. The display of the display unit 25 may include a touch panel. In a case where a part of the display unit 25 is formed of a touch panel, that part may also function as a user interface of the acquisition unit 23.

The user communicates with the server 30 by, for example, inputting a predetermined URL to the interface unit 20 or reading a QR code (registered trademark) by the interface unit 20. The user may start communication with the server 30 by selecting an icon displayed on the display unit 25.

The server 30 includes an information acquisition unit 31, an information output unit 32, an information analysis unit 33, and an information storage unit 34. The information acquisition unit 31 is configured to be able to acquire information. In one example, the information acquisition unit 31 acquires information from the communication unit 24 of the interface unit 20. The information output unit 32 is configured to be able to output information. In one example, the information output unit 32 outputs information to the communication unit 24 of the interface unit 20.

The information analysis unit 33 executes various analyses and controls. The information analysis unit 33 includes an arithmetic processing unit that executes a predetermined control program. The arithmetic processing unit includes, for example, a CPU or an MPU. The information analysis unit 33 is configured to be able to analyze the input information I from the user. In the first example, the information analysis unit 33 analyzes the input information I using the learning model M by machine learning. The input information I in this case includes the oral cavity image P of the user. In one example, the learning model M is a supervised learning model, which is one of the models of machine learning. In the second example, the information analysis unit 33 analyzes the input information I with reference to a correspondence table stored in the information storage unit 34. The input information I in this case includes information regarding oral care of the user instead of or in addition to the oral cavity image P. The correspondence table is a table in which the input information I and the analysis information A are associated with each other. In the correspondence table, the input information I and the analysis information A may be associated with the estimation information E. In a case where the input information I is further included in addition to the oral cavity image P, in one example, the learning model M further includes another learning model that analyzes the input information I other than the oral cavity image P. Another learning model is configured to be able to output, for example, a parameter for correcting the analysis information A of the oral cavity image P. In another example, the learning model M is configured as a model capable of executing multimodal learning for performing learning by combining both the oral cavity image P and the input information I not including the oral cavity image P.

The information storage unit 34 stores at least one of the learning model M, the correspondence table, and various types of information. The information analysis unit 33 refers to the learning model M, the correspondence table, and various types of information stored in the information storage unit 34 as necessary. The information storage unit 34 includes, for example, a nonvolatile memory and a volatile memory. The nonvolatile memory includes, for example, at least one of a ROM, an EPROM, an EEPROM, and a flash memory. The volatile memory includes, for example, a RAM.

The analysis of the input information I including the oral cavity image P using the learning model M executed by the information analysis unit 33 will be described. The step of analysis executed by the information analysis unit 33 includes a number of processes. The processes include a first process of detecting an oral region R from the oral cavity image P of the user, a second process of calculating the analysis information A by analyzing the oral region R included in the oral cavity image P, a third process of estimating the estimation information E from the analysis information A, and a fourth process of calculating the output information O corresponding to the analysis information A and the estimation information E. The acquisition of the estimation information E in the third process has an effect of improving the accuracy of the output information O.

The first process is executed by the information analysis unit 33 with any means. In one example, the oral region R is detected from the oral cavity image P of the oral cavity of the user using a learned model suitable for detecting the face. The oral region R includes a first oral region R1 including the teeth of the user's upper jaw and a second oral region R2 including the teeth of the user's lower jaw. The learned model for detecting the face is introduced into the information analysis unit 33 by, for example, the API.

In the first process, in a case in which the oral region R cannot be acquired from the oral cavity image P, the information analysis unit 33 outputs a content indicating the result to the display unit 25 of the interface unit 20 via the information output unit 32. In one example, when the oral region R cannot be recognized because the oral region R is not included in the oral cavity image P, the oral cavity image P is blurry, or the brightness of the oral cavity image P is not appropriate, the display unit 25 of the interface unit 20 outputs information requesting the user to again input the input information I including the oral cavity image P. In a case where one of the first oral region R1 and the second oral region R2 can be acquired and the other cannot be acquired, the information analysis unit 33 may determine that the first process has been completed, or may output, to the interface unit 20, information requesting the user to again input the input information I.

In the second process, the information analysis unit 33 analyzes the oral cavity image P. The information analysis unit 33 analyzes the oral cavity image P using the learning model M stored in the information storage unit 34. The analysis information A, which is the analysis result, includes at least one of information regarding presence or absence or a degree of interdental space, gingival recession, teeth crowding, gingival inflammation, insufficient brushing, bruxism, caries, hyperesthesia, bad breath, and coloring at the first specific site in the oral region R, and information regarding a state of teeth, gums, and oral mucosa corresponding to the first specific site. The information regarding the state of the teeth, gums, and oral mucosa includes at least one of, for example, the presence or absence or degree of tooth wear, tooth loss, and tooth fracture, color, dryness, and texture. The texture includes, for example, a plurality of textures including a hard texture that is felt to be firm when touched with a finger and a soft texture that is felt to be tender when touched with a finger. In a case where the results differ between the analysis information A in the first oral region R1 and the analysis information A in the second oral region R2, one of the two types of the analysis information A may be set in advance to be used. Alternatively, the two pieces of analysis information A may be quantified and an average thereof may be used. The two types of analysis information A may be quantified and the analysis information A having a larger absolute value may be used. Both the results of the analysis information A in the first oral region R1 and the analysis information A in the second oral region R2 may be used for the analysis.

In the third process, the information analysis unit 33 estimates the estimation information E from the input information I and the analysis information A. The estimation information E includes information on at least one of the presence or absence, the degree, and the probability of a predetermined state. The estimation information E includes at least one of information regarding presence or absence or a degree of interdental space, gingival recession, teeth crowding, gingival inflammation, insufficient brushing, bruxism, caries, hyperesthesia, bad breath, and coloring at the second specific site different from the first specific site, or information regarding a state of teeth, gums, and oral mucosa corresponding to the second specific site. The second specific site includes at least one of a premolar and a molar in the oral cavity. The estimation information E includes at least one of information on a premolar and information regarding a molar in the oral cavity. The estimation information E may include at least one of information regarding whether or not periodontal disease is present in the oral cavity of the user, information regarding whether or not chewing function is normal, information regarding whether or not occlusion is normal, and information regarding whether or not swallowing function is normal. The information on whether or not periodontal disease is present is information on at least one of the size, depth, and number of so-called periodontal pockets that are formed between the gingiva and the tooth. The information on whether or not the chewing function is normal is, for example, information on chewing force, presence or absence of unbalanced chewing, and chewing sound. The information on whether or not occlusion is normal is, for example, information on the biting force and the fitting of the tooth of the upper jaw with the tooth of the lower jaw. The information on whether or not the swallowing function is normal is, for example, information on presence or absence of swallowing difficulty.

In the fourth process, the information analysis unit 33 calculates the output information O corresponding to at least the input information I and the analysis information A. Preferably, the output information O is calculated with further reference to the estimation information E estimated from the analysis information A.

The learning model M for analyzing the oral region R will be described. The learning model M includes a plurality of learned models. The learning model M includes a first learned model M1 that determines whether or not the oral region R can be evaluated and a second learned model M2 that actually evaluates the oral region R.

The first learned model M1 includes at least one of a learning model M11 that determines whether or not the oral region R of the oral cavity image P has an analyzable image quality, a learning model M12 that determines whether or not the teeth crowding evaluation is possible, a learning model M13 that determines whether or not the interdental space evaluation of the teeth of the upper jaw is possible, a learning model M14 that determines whether or not the interdental space evaluation of the teeth of the lower jaw is possible, and a learning model M15 that determines whether or not the gingival recession evaluation is possible.

The second learned model M2 includes at least one of a learning model M21 that determines presence or absence of teeth crowding in the tooth of the upper jaw, a learning model M22 that determines presence or absence of teeth crowding in the tooth of the lower jaw, a learning model M23 that determines presence or absence of an interdental space in the tooth of the upper jaw, a learning model M24 that determines presence or absence of an interdental space in the tooth of the lower jaw, and a learning model M25 that determines presence or absence of interdental recession.

The creation of the learning model M will be described. The learning model M was created based on approximately 10,000 oral cavity images P. The developer classified the oral cavity image P into a learning image, a verification image, and a test image. The learning image is supervised data at the time of creating the learning model M. The verification image is an image for correcting the operation of the learning model M based on the learning image. The test image is an image for finally confirming the operation of the learning model M. The test image is used, for example, to determine whether or not the learning model M is causing overfitting. The learning image accounts for about 56% of the whole. The verification image accounts for about 24% of the whole. The test image accounts for 20% of the whole. In the present embodiment, the learning model M outputs a result of class classification. The learning model M outputs at least one of information regarding presence or absence or a degree of interdental space, gingival recession, teeth crowding, gingival inflammation, insufficient brushing, bruxism, caries, hyperesthesia, and coloring, information regarding a state of teeth, gums, and oral mucosa corresponding to the second specific site, information regarding whether or not periodontal disease is present in the oral cavity of the user, information regarding whether or not chewing force is lowering, information regarding whether or not biting force is lowering, and information regarding whether or not bad breath occurred.

Fig. 2 illustrates an example of the oral cavity image P of the user. The oral cavity image P includes at least a first specific site in the oral cavity. The first specific site includes the central incisor T1, the gum corresponding to the central incisor T1, the lateral incisor T2, the gum corresponding to the lateral incisor T2, the canine T3, and the gum corresponding to the canine T3. The oral cavity image P includes a tooth image and a gum image in the oral cavity. The tooth image includes an image of the central incisor T1, an image of the lateral incisor T2, and an image of the canine T3 in the oral cavity. The gum image includes images of the gums corresponding to the central incisor T1, the lateral incisor T2, and the canine T3. The oral cavity image P includes at least one of the four first specific sites located at upper or lower and left or right positions in the oral cavity of the user.

A preferable range of the tooth image included in the oral cavity image P will be described with reference to Fig. 3. In the determination of the presence or absence of the teeth crowding, it is preferred that the oral cavity image P includes a range from the end portion E1 of the central incisor T1 to the broken line L2. In the determination of the presence or absence of the interdental space, it is preferred that the oral cavity image P includes a range from the end portion E1 of the central incisor T1 to the double-dashed line L3. In the determination of the presence or absence of gingival recession, it is preferred that the oral cavity image P includes a range from the end portion E1 of the central incisor T1 to the solid line L1. When the oral cavity image P includes the image of the central incisor T1 in each range described above, the oral cavity image P includes the image of the corresponding gum.

Each oral cavity image P was supervised by an expert who analyzes the oral cavities. Figs. 4 to 9 each illustrate an example of a supervised oral cavity image P. An expert who analyzes oral cavities is, for example, a dentist, a dental hygienist, a researcher who studies oral states, or a developer who develops oral care items. An expert who analyzes oral cavities performed a determination of the degree of teeth crowding, a determination of the degree of gingival recession, and a determination of the degree of interdental space for each of the first oral region R1 and the second oral region R2 in the oral cavity image P of the oral cavity. In the determination of the degree of teeth crowding, the expert who analyzes the oral cavities performed a determination based on the type of teeth crowding. In the determination of gingival recession, the expert who analyzes oral cavities performed a determination of a plurality of stages from no gingival recession to complete gingival recession. In the determination of the degree of interdental space, the expert who analyzes oral cavities performed an evaluation of a plurality of stages from no interdental space to severe interdental space with respect to each of the upper jaw and the lower jaw.

The state of the oral cavity at the first specific site is correlated with the state of the oral cavity at the second specific site different from the first specific site. In one example, a test conducted on seventy-five women in their 40's to 70's shows that the state of the gingiva in the front tooth and the state of the gingiva in the back tooth are highly correlated. That is, when the gingival recession occurs at the first specific site, the gingival recession also occurs at the second specific site. The same applies to the degree of interdental space and teeth crowding.

Calculation of the analysis information A executed by the information analysis unit 33 in a case where information regarding oral care is included will be described. In this case, the output information O further includes answer information. The answer information includes an example of an appropriate oral care for the user based on the information regarding oral care. As a first example, a case where the information regarding oral care includes information regarding an oral care item using action of the user will be described. The information analysis unit 33 compares the information regarding the oral care item using action with the information stored in the information storage unit 34. For example, in a case where at least one of the frequency of use, the number of times used, and the using time of the oral care item is less than a predetermined value stored in the information storage unit 34, the information analysis unit 33 outputs answer information for prompting the user to take an action so that the value becomes greater than the predetermined value. The predetermined value may be correctable based on, for example, the analysis information A or the estimation information E. For example, in a case where the analysis information A or the estimation information E includes information that teeth crowding is present in the oral cavity, the information analysis unit 33 changes to increase the predetermined value of at least one of the frequency of use, the number of times used, and the using time of the oral care item. As a second example of the user, a case where the information regarding oral care includes information regarding a lifestyle of the user will be described. The information analysis unit 33 compares the information regarding the lifestyle of the user with the information stored in the information storage unit 34. For example, in a case where a food with a high sugar content is frequently taken in the dietary life, the information analysis unit 33 outputs answer information for prompting the user to take an action so as to increase the number of times and time of oral care. As a third example, a case where the information regarding oral care includes information regarding intraoral endocrine of the user will be described. The information analysis unit 33 compares the acidity of the intraoral endocrine of the user with a standard acidity of intraoral endocrine. When the difference in acidity is greater than or equal to the predetermined value, the information analysis unit 33 outputs recommendation information RI on an oral washing agent suitable for reducing the difference in acidity and answer information on an appropriate frequency of use of the oral washing agent.

The information analysis unit 33 outputs the analysis information A, the estimation information E, and the answer information to a predetermined configuration. In the first example, the information analysis unit 33 outputs information to the information output unit 32. In the second example, the information analysis unit 33 outputs information to the information storage unit 34. In the third example, the information analysis unit 33 outputs information to both the information output unit 32 and the information storage unit 34.

The information analysis unit 33 associates at least the analysis information A with the output information O. Preferably, the information analysis unit 33 further includes the input information I and the estimation information E and associates the input information I and the estimation information E with the output information O. The output information O will be described. The output information O includes recommendation information RI regarding oral care of the user. The recommendation information RI includes at least oral care item recommendation information RI1 for recommending an oral care item in accordance with the oral state of the user. The recommendation information RI may further include information RI2 regarding usage method of the oral care item. The information RI2 regarding the usage method of the oral care item may be a usage method of the oral care item included in the oral care item recommendation information RI1, or may be a general usage method of an oral care item that is not included in the oral care item recommendation information RI1. The recommendation information RI may further include information regarding a recommendation for a visit to a dental office, a recommendation for a treatment at a dental office, and a recommendation for improvement of lifestyle. The treatment is, for example, treatment of periodontal disease, implementation of implants, and orthodontics. Improvement in lifestyle is, for example, a change in eating time and a reduction in smoking habit.

The correspondence of the analysis information A, the estimation information E, and the answer information with the output information O is executed by the information analysis unit 33 with any means. In the first example, it is executed using a correspondence table in which the analysis information A, the estimation information E, and the answer information are associated with the output information O in advance by a researcher who studies oral states or a developer who develops oral care items. In the second example, the correspondence between the analysis information A and the output information O is executed using a model of machine learning.

The output information O includes a plurality of pieces of output information. In one example, the output information O includes first output information O1, second output information O2, third output information O3, fourth output information O4, and fifth output information O5. Each piece of the output information O1 to O5 includes different types of recommendation information RI.

The first output information O1 is output information O in a case where there is teeth crowding in the oral cavity of the user. The first output information O1 includes information on a toothbrush having a small head size as the oral care item recommendation information RI1. The toothbrush having a small head size is, for example, a tuft brush. The information RI2 related to the usage method of the oral care item includes, for example, a brushing operation in a pen gripping manner.

The second output information O2 is output information O in a case where there is no teeth crowding in the oral cavity of the user, there is gingival inflammation, and there is gingival recession. The second output information O2 includes, as the oral care item recommendation information RI1, information on a product having a brush hardness that is a first hardness. The information RI2 related to the usage method of the oral care item includes, for example, a brushing operation in a pen gripping manner.

The third output information O3 is output information O in a case where there is no teeth crowding in the oral cavity of the user, there is gingival inflammation, and there is no gingival recession. The third output information O3 includes, as the oral care item recommendation information RI1, information on a product having a brush hardness that is a second hardness. The information RI2 related to the usage method of the oral care item includes, for example, a brushing operation in a palm gripping manner. The second hardness is about the same as the first hardness.

The fourth output information O4 is output information O in a case where there is no teeth crowding in the oral cavity of the user, there is no gingival inflammation, and there is gingival recession. The fourth output information O4 includes, as the oral care item recommendation information RI1, information on a product having a brush hardness that is a third hardness. The information RI2 related to the usage method of the oral care item includes, for example, a brushing operation in a pen gripping manner. The third hardness is greater than the first hardness and the second hardness.

The fifth output information O5 is output information O in a case where there is no teeth crowding in the oral cavity of the user, there is no gingival inflammation, and there is no gingival recession. The fifth output information O5 includes, as the oral care item recommendation information RI1, information on a product having a brush hardness that is a fourth hardness. The information RI2 related to the usage method of the oral care item includes, for example, a brushing operation in a pen gripping manner. The fourth hardness is greater than the third hardness.

In at least one of the pieces of the output information O1 to O5, the recommendation information RI may further include a different piece of oral care item recommendation information RI1. In one example, information is added depending on the presence or absence of an interdental space. In a case where determination is made that there is an interdental space in the oral cavity by analysis of the oral cavity image P or input by the user, the oral care item recommendation information RI1 further includes, for example, information regarding an appropriate interdental brush. The information on the appropriate interdental brush is further modified in accordance with the degree of interdental space. When determination is made that there is no interdental space, the oral care item recommendation information RI1 further includes, for example, information regarding appropriate dental floss. In another example, the oral care item recommendation information RI1 includes oral care item recommendation information RI1 that recommends a toothbrush having a smaller head size when it is determined that the toothbrush is finely moved than when it is determined that the toothbrush is not finely moved.

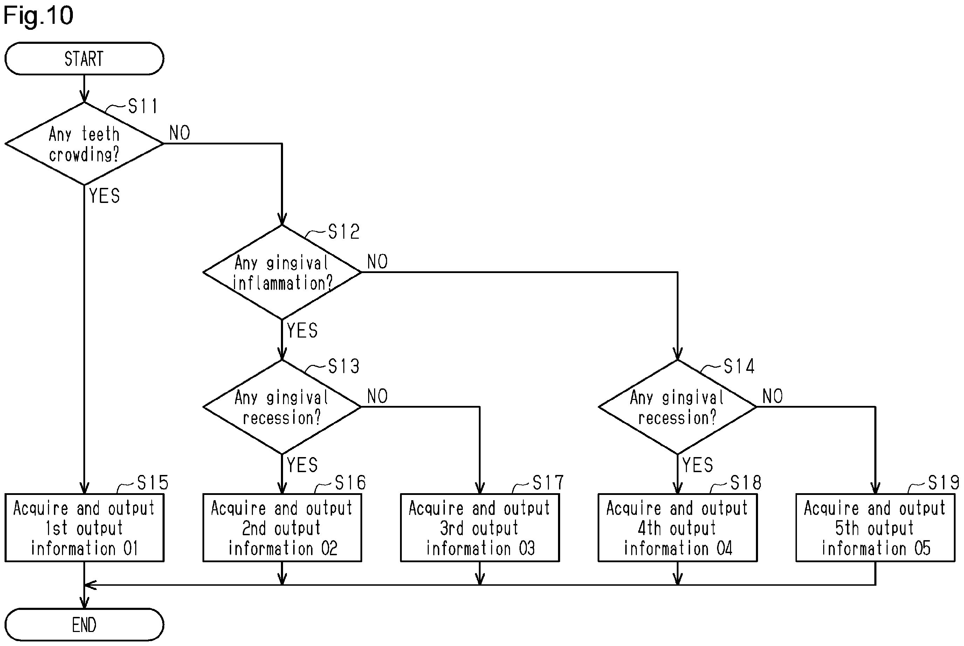

An example of correspondence and output control of the output information O executed by the information analysis unit 33 will be described with reference to Fig. 10. The information analysis unit 33 executes the correspondence with the output information O and outputs the output information O, for example, triggered by the acquisition of the analysis information A and the estimation information E.

In the process of step S11, the information analysis unit 33 refers to the analysis information A and the estimation information E to determine whether or not there is teeth crowding in the oral cavity. In the case of an affirmative determination, the information analysis unit 33 executes the process of step S15. In the case of a negative determination, the information analysis unit 33 executes the process of step S12.

In the process of step S12, the information analysis unit 33 refers to the analysis information A and the estimation information E to determine whether or not there is gingival inflammation in the oral cavity. In the case of an affirmative determination, the information analysis unit 33 executes the process of step S13. In the case of a negative determination, the information analysis unit 33 executes the process of step S14.

In the process of step S13, the information analysis unit 33 refers to the analysis information A and the estimation information E and determines whether or not there is gingival recession in the oral cavity. In the case of an affirmative determination, the information analysis unit 33 executes the process of step S16. In the case of a negative determination, the information analysis unit 33 executes the process of step S17.

In the process of step S14, the information analysis unit 33 refers to the analysis information A and the estimation information E and determines whether or not there is gingival recession in the oral cavity. In the case of an affirmative determination, the information analysis unit 33 executes the process of step S18. In the case of a negative determination, the information analysis unit 33 executes the process of step S19.

In the process of step S15, the information analysis unit 33 outputs the first output information O1. The first output information O1 is output to, for example, the display unit 25. After the end of the process of step S15, the control ends.

In the process of step S16, the information analysis unit 33 outputs the second output information O2. The second output information O2 is output to, for example, the display unit 25. After the end of the process of step S16, the control ends.

In the process of step S17, the information analysis unit 33 outputs the third output information O3. The third output information O3 is output to, for example, the display unit 25. After the end of the process of step S17, the control ends.

In the process of step S18, the information analysis unit 33 outputs the fourth output information O4. The fourth output information O4 is output to, for example, the display unit 25. After the end of the process of step S18, the control ends.

In the process of step S19, the information analysis unit 33 outputs the fifth output information O5. The fifth output information O5 is output to, for example, the display unit 25. After the end of the process of step S19, the control ends.

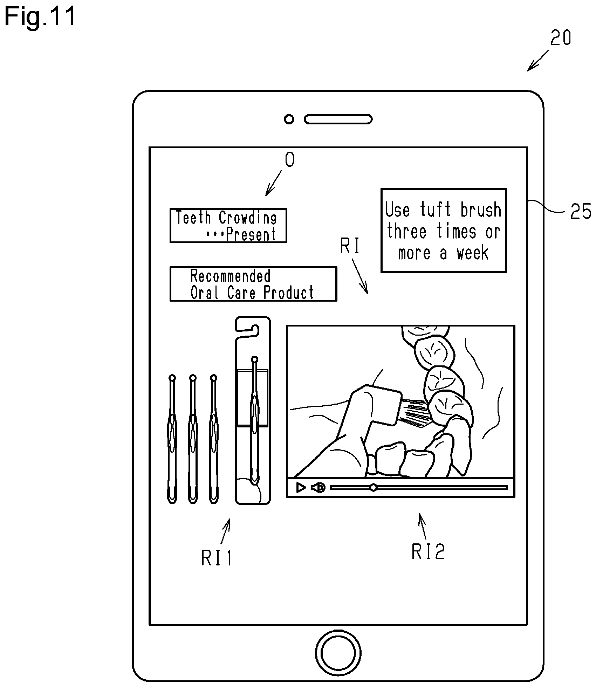

Fig. 11 illustrates an example of the output information O displayed on the display unit 25 of the interface unit 20. The interface unit 20 is a smartphone, and the display unit 25 is a display. The output information O is the first output information O1. The oral care item recommendation information RI1 is indicated by, for example, an image. The information RI2 regarding the usage method of the oral care item is indicated by, for example, a moving image.

The oral care item recommendation information RI1 includes at least one of a toothbrush, an interdental cleaning tool, and an oral washing agent. Preferably, the toothbrush, the interdental cleaning tool, and the oral washing agent include information related to a model number that specifies the type of each item. The oral care item recommendation information RI1 may include information regarding a store where a recommended item is obtained.

In one example, the information RI2 regarding the usage method of the oral care item displays a general usage method of the oral care item included in the oral care item recommendation information RI1 by a moving image. For example, when there is teeth crowding in the oral cavity, a brushing operation suitable to a place where teeth crowding is present is displayed by a moving image. In another example, a usage method of the oral care item at the first specific site included in the oral cavity image P of the user is schematically displayed. For example, when there is interdental space in the oral cavity, a usage method of an appropriate oral care item for that place is displayed.

The display unit 25 further displays answer information. In one example, in a case where there is teeth crowding in the oral cavity of the user, a sentence prompting the user to perform brushing using a tuft brush included in the oral care item recommendation information three or more times a week is displayed. For each of a case where the user implements the content displayed in the answer information and a case where the user does not, information indicating a future state in the oral cavity may be further displayed. Purchase information for purchasing the above tuft brush may be included. In one example, the purchase information is information on a store where the corresponding oral care product can be purchased or tried. In another example, the purchase information is site information on a web that sells the corresponding oral care product.

The operation of the oral care recommendation system 10 of the present embodiment will be described. The user inputs the input information I to the acquisition unit 23. The control unit 21 controls the communication unit 24 to output the input information I to the server 30. The server 30 acquires the input information I with the information acquisition unit 31. The information analysis unit 33 analyzes the input information I and calculates the analysis information A. The information analysis unit 33 estimates the estimation information E from the analysis information A. The information analysis unit 33 calculates the output information O including the recommendation information RI from the input information I, the analysis information A, and the estimation information E. The server 30 outputs the output information O from the information output unit 32 to the interface unit 20. The control unit 21 acquires the output information O from the communication unit 24 and displays the output information O on the display unit 25. This allows the user to recognize the recommendation information RI included in the output information O via the display of the display unit 25.

### Modification of Second Embodiment

The description related to the second embodiment exemplifies applicable forms of an oral care recommendation system according to the present disclosure and is not intended to limit the forms. The present disclosure may take, other than the embodiment, a form in which, for example, modifications of the embodiment shown below and at least two modifications that do not contradict each other are combined.

The learning model M may be configured to output a result of regression analysis. In this case, the learning model M quantifies and outputs at least one of the degree of teeth crowding, the degree of interdental space, and the degree of gingival recession.

At least one of the learned model M1 and the learned model M2 may be a model learned by unsupervised learning or reinforcement learning. At least one of the learning models M11 to M15 and the learning models M21 to M25 may be a model learned by unsupervised learning or reinforcement learning.

The learning model M may include a first learned model M1 that determines whether or not evaluation is possible and a second learned model M2 that performs evaluation without distinguishing the first oral region R1 and the second oral region R2 in the oral cavity image P. The learning model M may include a first learned model M1 that recognizes a set of a tooth and gum in the oral cavity image P and determines whether or not evaluation is possible and a second learned model M2 that performs evaluation. In addition, the learning model M may include a learning model M that recognizes a set of a tooth and gum in the oral region R and executes analysis when a majority of the teeth and gums can be evaluated. In addition, the analysis information A of each set of a tooth and gum may be set in advance to be used. The analysis information A of each set of a tooth and gum may be quantified and an average thereof may be used, or the analysis information A of each set of a tooth and gum may be quantified and the analysis information A having a large absolute value may be used.

The learning model M may be configured to display the oral cavity image P as the output information O in a pseudo color. In one example, a region used for analysis using the learning model M in the oral cavity image P or a region having interdental space, gingival recession, or teeth crowding are displayed in red. The user can easily recognize the region used for the analysis and the region having a problem in the oral state.

The analysis information A may include the degree of stain of teeth at the first specific site. The information analysis unit 33 performs analysis using a learning model for analyzing stain of teeth at the first specific site. The estimation information E includes information on the degree of stain of teeth at the second specific site.

The output information O may further include at least one of information regarding a current oral state of the user, information regarding prediction of a future oral state of the user, and information regarding a health state of the user affected by the oral state of the user. The information regarding the current oral state of the user includes at least one of information regarding presence or absence or a degree of interdental space, gingival recession, teeth crowding, gingival inflammation, insufficient brushing, bruxism, caries, hyperesthesia, bad breath, and coloring, information regarding whether or not periodontal disease is present, information regarding whether or not chewing function is normal, information regarding whether or not occlusion is normal, information regarding whether or not swallowing function is normal, and information regarding a state of teeth, gums, and oral mucosa corresponding to the second specific site. The information regarding prediction of the future oral cavity of the user includes an estimated image showing an oral state of the user after a predetermined period has elapsed. The information regarding the health state of the user affected by the oral state of the user includes, for example, information regarding a health state other than the oral cavity related to the periodontal disease or the oral state.

The oral cavity image P may be a moving image in which movements in and around the oral cavity are captured, or a moving image including movements when the user uses the oral care item. The oral cavity image P may be an image including both the first specific site and the second specific site in the oral cavity, or may be an image including only the second specific site.

The answer information may be configured further based on the analysis information A of the oral cavity image P. In one example, the oral cavity image P is a moving image including movement when the user uses the oral care item. The answer information includes information on a more appropriate usage method from the usage method of the oral care item of the user included in the oral cavity image P. For example, in a case where the number of times the user uses the oral care item between the central incisor T1 and the lateral incisor T2 is small and the time of each use is short, resulting in insufficient brushing, the answer information includes information prompting an action of care between the central incisor T1 and the lateral incisor T2.

The input information I may be acquired via the loT device. In one example, the input information I is acquired by connecting an loT device to an oral care item used for brushing. In one example, the input information I includes information on the number of times teeth are brushed in a day, and the frequency of use of the oral care item. The loT device may transmit the input information I to the acquisition unit 23, or may transmit the input information I to the server 30.

At least one of the learning model M, the correspondence table, and various types of information may be stored in a place other than the information storage unit 34 of the server 30. In one example, it is stored in a storage unit provided in the interface unit 20. In another example, it is stored in a database configured in an external environment.

The input information I may include first input information 11 including user's current state information and second input information I2 being user's past state information. In a case where the first input information 11 and the second input information I2 include the oral cavity image P of the user, the oral cavity image P included in the second input information I2 is an image of the oral cavity of the user taken at a predetermined time before the first input information 11 is acquired. In a case where the first input information I1 and the second input information I2 include information regarding oral care of the user, the information regarding oral care included in the second input information I2 is information regarding oral care a predetermined time before the first input information I1 is acquired. The predetermined time is, for example, a time of one month or more. In another example, the predetermined time is a time of one year or more. The second input information I2 may be transmitted to the server 30 via the communication unit 24 before the acquisition unit 23 acquires the first input information I1, or may be transmitted to the server 30 via the communication unit 24 simultaneously with the transmission of the first input information I1. In this case, the interface unit 20 further includes a storage unit configured to store at least the second input information I2. The input information I may further include third input information I3 acquired a predetermined time before acquisition of the second input information I2. The information analysis unit 33 calculates the analysis information A and the estimation information E using at least one of the pieces of the first input information I1 to the third input information I3. The accuracy is further improved by adding the third input information I3. The interval of the predetermined time may be changed by each piece of input information I. The input information I may further include input information I including fourth input information and so on.

### Example of Applicable Form of Oral State Evaluation System in Third Embodiment

(3-1) An oral state evaluation system according to the present disclosure includes an information acquisition unit configured to acquire an oral cavity image including at least a first specific site in an oral cavity as input information from an interface unit; and an information analysis unit configured to analyze the input information by a learning model that has learned the oral cavity image in advance to evaluate the state in the oral cavity; where the information analysis unit estimates estimation information regarding a state at a second specific site in the oral cavity different from the first specific site.

In the oral state evaluation system described above, the information analysis unit estimates the estimation information regarding the state of the second specific site in the oral cavity different from the first specific site from the oral cavity image including the first specific site. The oral state of the entire oral cavity can be evaluated regardless of whether or not the oral cavity image includes the second specific site. Therefore, the oral state can be evaluated by a simple means.

(3-2) According to one example of the oral state evaluation system, the oral cavity image includes a tooth image and a gum image in the oral cavity.

According to the oral state evaluation system described above, since the oral cavity image includes an image of an appropriate site in the oral cavity, the oral state can be appropriately evaluated.

(3-3) According to one example of the oral state evaluation system, the tooth image includes a central incisor image, a lateral incisor image, and a canine image in the oral cavity.

According to the oral state evaluation system described above, since the oral cavity image includes an image of an appropriate tooth in the oral cavity, the oral state can be appropriately evaluated.

(3-4) According to one example of the oral state evaluation system, the second specific site includes at least one of a premolar and a molar in the oral cavity, and the estimation information includes at least one of information regarding the premolar and the molar in the oral cavity.

According to the oral state evaluation system described above, since at least one of information regarding the premolar and information regarding the molar in the oral cavity is estimated, the oral state can be appropriately evaluated.

(3-5) According to one example of the oral state evaluation system, the estimation information includes at least one of information regarding presence or absence or a degree of interdental space, gingival recession, teeth crowding, gingival inflammation, insufficient brushing, bruxism, caries, hyperesthesia, bad breath, and coloring at the second specific site, information regarding whether or not periodontal disease is present, information regarding whether or not chewing function is normal, information regarding whether or not occlusion is normal, information regarding whether or not swallowing function is normal, and information regarding a state of teeth, gums, and oral mucosa corresponding to the second specific site.

According to the oral state evaluation system described above, at least one of the states of the tooth and the gum at the second specific site can be estimated. Therefore, the oral state can be appropriately evaluated.

(3-6) One example of the oral state evaluation system further includes an information storage unit configured to store the learning model.

According to the oral state evaluation system described above, the learning model for analyzing the oral state can be suitably referenced.

(3-7) According to one example of the oral state evaluation system, the input information further includes at least one of information regarding a lifestyle of a user, information regarding an intraoral endocrine of the user, information regarding an intraoral bacterial flora of the user, information regarding an attribute of the user, and information obtained by a sensor that detects a state in the oral cavity.

According to the oral state evaluation system described above, the oral state can be more appropriately evaluated.

(3-8) One example of the oral state evaluation system further includes an information output unit configured to output information corresponding to the estimation information as output information, where the information output unit outputs the output information to at least the interface unit.

According to the oral state evaluation system described above, the user can easily recognize the output information.

(3-9) According to one example of the oral state evaluation system, the output information includes at least one of information regarding a current oral state of a user, information regarding prediction of a future oral state of the user, information regarding an oral care method for the user, and information regarding a health state of the user affected by the oral state of the user.

According to the oral state evaluation system described above, the user can suitably recognize the output information related to the oral state.

### Third Embodiment

An oral state evaluation system 10 according to a third embodiment will be described with reference to Figs. 1 to 9. The oral state evaluation system 10 is a system that acquires and analyzes input information I and estimates estimation information E. The oral state evaluation system 10 may calculate output information O corresponding to the estimation information E, and output the output information O to a predetermined configuration. A main element forming the oral state evaluation system 10 is a server 30. Preferably, the oral state evaluation system 10 includes an interface unit 20 for exchanging information with the server 30. In one example, the interface unit 20 is a smart device configured to be carried by the user. The smart device includes a tablet terminal or a smartphone. In another example, the interface unit 20 is a personal computer. The personal computer is installed at the user's residence, store, or dental office. A store includes a store that sells oral care items or a store that sells other items. The interface unit 20 and the server 30 are configured to be able to communicate with each other using, for example, the Internet connection N. In another example, the interface unit 20 and the server 30 are integrally configured.

The interface unit 20 includes a control unit 21, a storage unit 22, an acquisition unit 23, a communication unit 24, and a display unit 25. The control unit 21 includes an arithmetic processing unit that executes a predetermined control program. The arithmetic processing unit includes, for example, a CPU, a GPU, or an MPU.

The storage unit 22 stores various control programs executed by the control unit 21 and information used for various control processes. The storage unit 22 includes, for example, a nonvolatile memory and a volatile memory. The nonvolatile memory includes, for example, at least one of a ROM, an EPROM, an EEPROM, and a flash memory. The volatile memory includes, for example, a RAM.

The acquisition unit 23 acquires the input information I from the user. The acquisition unit 23 has any configuration for acquiring the input information I of the user. In the first example, it is a camera capable of acquiring an oral cavity image P of the user's oral cavity. The oral cavity image P includes a still image and a moving image. The oral cavity image P includes a three-dimensional image having depth information or a panoramic image formed by joining a plurality of images. The camera may be a camera mounted on the smart device, or may be a separate independent camera. The independent camera is, for example, a pen type camera in which a camera is provided at a distal end portion suitable for photographing the oral cavity or a camera capable of photographing a range of 360°. The independent camera is configured to be capable of wired or wireless communication with the control unit 21. The oral cavity image P captured by the independent camera is transmitted to the control unit 21 by wired or wireless communication. In the second example, the acquisition unit 23 is a user interface configured to allow the user to input or select a character or the like. The input information I of the user may further include at least one of information regarding the lifestyle of the user, information regarding the intraoral endocrine of the user, information regarding the intraoral bacterial flora of the user, answer information to a predetermined question, and information regarding an attribute of the user. The information regarding the lifestyle of the user includes information regarding the user's meal content, oral caring action, and wake-up or sleeping time. The information regarding the intraoral endocrine of the user includes information on the amount of saliva, viscosity, hydrogen ion concentration, amount of antimicrobial component, and amount of component related to tooth remineralization. The information regarding the intraoral bacterial flora of the user includes information regarding the amount and type of main bacteria present in saliva, dental plaque, or mucosa, and information regarding the type and amount of antibodies of the main bacteria. The information regarding the intraoral endocrine of the user and the information regarding the intraoral bacterial flora of the user may be a result of an interview with the user, or may be result information obtained by examining the intraoral endocrine or the like existing in the oral cavity of the user by a predetermined means. The answer information regarding the predetermined question includes a question about the state of the user's gums, a preferred brushing method during oral care, the number of times teeth are brushed in a day, the time taken for brushing teeth each time, the time at which teeth are brushed, and the presence or absence of dentures. The question regarding the state of the user's gums includes, for example, the presence or absence of bleeding from the gums at the time of brushing or eating or drinking. The information regarding the attribute of the user is, for example, the user's own age, sex, height, weight, dominant arm, and medical history. In the third example, the acquisition unit 23 is a sensor that detects a state in the oral cavity. The sensor is, for example, a fluorescent sensor and a temperature sensor. The fluorescence sensor emits light of a predetermined wavelength, quantifies the amount of light, and measures the distribution and amount of a predetermined object in the oral cavity. The predetermined object is, for example, stain or plaque. The temperature sensor measures a temperature in the oral cavity. The sensor is configured to be capable of wired or wireless communication with the control unit 21. Information obtained by the sensor is transmitted to the control unit 21 by wired or wireless communication. In the present embodiment, the acquisition unit 23 may be configured by combining two or more of the first to third examples. The sensor may be further configured to be able to measure at least one of chewing force, biting force, amount of bleeding from gums, bad breath, strength of brush pressure at the time of brushing, or movement of toothbrush at the time of brushing.

The communication unit 24 is configured to be able to communicate with the outside of the interface unit 20 based on the control of the control unit 21. The communication unit 24 is configured to be able to communicate via the Internet connection N. The communication unit 24 may be configured to be able to communicate with the server 30 of the interface unit 20 through wired communication or wireless communication. The communication unit 24 transmits, for example, the input information I of the user acquired by the acquisition unit 23 based on the control of the control unit 21, and receives the output information O from the server 30.

The display unit 25 displays various types of information based on the control of the control unit 21. The various types of information are, for example, information regarding the input information I of the user and information regarding the output information O from the server 30. In one example, the display unit 25 includes a display. The display of the display unit 25 may include a touch panel. In a case where a part of the display unit 25 is formed of a touch panel, that part may also function as a user interface of the acquisition unit 23.

The user communicates with the server 30 by, for example, inputting a predetermined URL to the interface unit 20 or reading a QR code (registered trademark) by the interface unit 20. The user may start communication with the server 30 by selecting an icon displayed on the display unit 25.

The server 30 includes an information acquisition unit 31, an information output unit 32, an information analysis unit 33, and an information storage unit 34. The information acquisition unit 31 is configured to be able to acquire information. In one example, the information acquisition unit 31 acquires information from the communication unit 24 of the interface unit 20. The information output unit 32 is configured to be able to output information. In one example, the information output unit 32 outputs information to the communication unit 24 of the interface unit 20.

The information analysis unit 33 executes various analyses and controls. The information analysis unit 33 includes an arithmetic processing unit that executes a predetermined control program. The arithmetic processing unit includes, for example, a CPU or an MPU. The information analysis unit 33 is configured to be able to analyze the input information I from the user. In the first example, the information analysis unit 33 analyzes the input information I using the learning model M by machine learning. The input information I in this case includes the oral cavity image P of the user. In one example, the learning model M is a supervised learning model, which is one of the models of machine learning. In the second example, the information analysis unit 33 analyzes the input information I with reference to a correspondence table stored in the information storage unit 34. The input information I in this case includes, instead of or in addition to the oral cavity image P, at least one of information regarding the lifestyle of the user, information regarding the user's intraoral endocrine, information regarding the user's intraoral bacterial flora, and answer information to a predetermined question. The correspondence table is a table in which at least one of the input information I or the estimation information E is associated with the output information O. In a case where the input information I is further included in addition to the oral cavity image P, in one example, the learning model M further includes another learning model that analyzes the input information I other than the oral cavity image P. Another learning model is configured to be able to output, for example, a parameter for correcting the estimation information E of the oral cavity image P. In another example, the learning model M is configured as a model capable of executing multimodal learning for performing learning by combining both the oral cavity image P and the input information I not including the oral cavity image P.

The information storage unit 34 stores at least one of the learning model M, the correspondence table, and various types of information. The information analysis unit 33 refers to the learning model M, the correspondence table, and various types of information stored in the information storage unit 34 as necessary.

The analysis of the input information I including the oral cavity image P using the learning model M executed by the information analysis unit 33 will be described. The step of analysis executed by the information analysis unit 33 includes a number of processes. The processes include a first process of detecting the oral region R from the oral cavity image P of the user, a second process of estimating the estimation information E from the input information I, and a third process of calculating the output information O corresponding to the estimation information E.

The first process is executed by the information analysis unit 33 with any means. In one example, the oral region R is detected from the oral cavity image P of the oral cavity of the user using a learned model suitable for detecting the face. The oral region R includes a first oral region R1 including the teeth of the user's upper jaw and a second oral region R2 including the teeth of the user's lower jaw. The learned model for detecting the face is introduced into the information analysis unit 33 by, for example, the API.

In the first process, in a case where the oral region R cannot be acquired from the oral cavity image P, the information analysis unit 33 outputs a content indicating the result to the display unit 25 of the interface unit 20 via the information output unit 32. In one example, when the oral region R cannot be recognized because the oral region R is not included in the oral cavity image P, the oral cavity image P is blurry, or the brightness of the oral cavity image P is not appropriate, the display unit 25 of the interface unit 20 outputs information requesting the user to again input the input information I including the oral cavity image P. In a case where one of the first oral region R1 and the second oral region R2 can be acquired and the other cannot be acquired, the information analysis unit 33 may determine that the first process has been completed, or may output, to the interface unit 20, information requesting the user to again input the input information I.

In the second process, the information analysis unit 33 analyzes the oral cavity image P using the learning model M stored in the information storage unit 34 and estimates the estimation information E. The estimation information E includes information on at least one of the presence or absence, the degree, and the probability of a predetermined state. The estimation information E includes at least one of information regarding presence or absence or a degree of interdental space, gingival recession, teeth crowding, gingival inflammation, insufficient brushing, bruxism, caries, hyperesthesia, bad breath, and coloring at the second specific site different from the first specific site, or information regarding a state of teeth, gums, and oral mucosa corresponding to the second specific site. The information regarding the state of the teeth, gums, and oral mucosa includes at least one of, for example, the presence or absence or degree of tooth wear, tooth loss, and tooth fracture, color, dryness, and texture. The texture includes, for example, a plurality of textures including a hard texture that is felt to be firm when touched with a finger and a soft texture that is felt to be tender when touched with a finger. The second specific site includes at least one of a premolar and a molar in the oral cavity. The estimation information E includes at least one of information on a premolar and information regarding a molar in the oral cavity. The estimation information E may include at least one of information regarding whether or not periodontal disease is present in the oral cavity of the user, information regarding whether or not chewing function is normal, information regarding whether or not occlusion is normal, and information regarding whether or not swallowing function is normal. The information on whether or not periodontal disease is present is information on at least one of the size, depth, and number of so-called periodontal pockets that are formed between the gingiva and the tooth. The information on whether or not the chewing function is normal is, for example, information on chewing force, presence or absence of unbalanced chewing, and chewing sound. The information on whether or not occlusion is normal is, for example, information on the biting force and the fitting of the tooth of the upper jaw with the tooth of the lower jaw. The information on whether or not the swallowing function is normal is, for example, information on presence or absence of swallowing difficulty.

In the second process, the learning model M further calculates a correspondence between the first information and the second information. The first information is, for example, the presence or absence of teeth crowding at the first specific site. The second information is, for example, the presence or absence of an interdental space at the second specific site. The information analysis unit 33 calculates, for example, the relevance between the first information and the second information by statistical analysis. In a case where there is teeth crowding at the first specific site, if the statistical analysis using the learning model M defines that there is a correlation with respect to the existence of the interdental space at the second specific site, the information analysis unit 33 adds such information to the estimation information E. In another example, the first information is information regarding a user's dominant arm. The second information is presence or absence of gingival recession at the first specific site. According to the analysis using the learning model M, for example, in a case where the degree of gingival recession on the same side as the dominant arm is greater than the degree of gingival recession on the opposite side to the dominant arm at the first specific site, the care method of the user may be uneven. Thus, the information analysis unit 33 adds such information to the estimation information E.

In the third process, the information analysis unit 33 calculates the output information O corresponding to at least one of the estimation information E. The output information O includes at least one of information regarding a current oral state of a user, information regarding prediction of a future oral state of the user, information regarding an oral care method for the user, and information regarding a health state of the user affected by the oral state of the user.

The learning model M for analyzing the oral region R will be described. The learning model M includes a plurality of learned models. The learning model M includes a first learned model M1 that determines whether or not the oral region R can be evaluated and a second learned model M2 that actually evaluates the oral region R.

The first learned model M1 includes at least one of a learning model M11 that determines whether or not the oral region R of the oral cavity image P has an analyzable image quality, a learning model M12 that determines whether or not the teeth crowding evaluation is possible, a learning model M13 that determines whether or not the interdental space evaluation of the teeth of the upper jaw is possible, a learning model M14 that determines whether or not the interdental space evaluation of the teeth of the lower jaw is possible, and a learning model M15 that determines whether or not the gingival recession evaluation is possible.

The second learned model M2 includes at least one of a learning model M21 that determines presence or absence of teeth crowding in the tooth of the upper jaw, a learning model M22 that determines presence or absence of teeth crowding in the tooth of the lower jaw, a learning model M23 that determines presence or absence of an interdental space in the tooth of the upper jaw, a learning model M24 that determines presence or absence of an interdental space in the tooth of the lower jaw, and a learning model M25 that determines presence or absence of interdental recession.

The creation of the learning model M will be described. The learning model M was created based on approximately 10,000 oral cavity images P. The developer classified the oral cavity image P into a learning image, a verification image, and a test image. The learning image is supervised data at the time of creating the learning model M. The verification image is an image for correcting the operation of the learning model M based on the learning image. The test image is an image for finally confirming the operation of the learning model M. The test image is used, for example, to determine whether or not the learning model M is causing overfitting. The learning image accounts for about 56% of the whole. The verification image accounts for about 24% of the whole. The test image accounts for 20% of the whole. In the present embodiment, the learning model M outputs a result of class classification. The learning model M outputs at least one of information regarding presence or absence or a degree of interdental space, gingival recession, teeth crowding, gingival inflammation, insufficient brushing, bruxism, caries, hyperesthesia, bad breath, and coloring, information regarding whether or not periodontal disease is present in the oral cavity of the user, information regarding whether or not chewing function is normal, information regarding whether or not occlusion is normal, information regarding whether or not swallowing function is normal, and information regarding a state of teeth, gums, and oral mucosa corresponding to the second specific site.

Fig. 2 illustrates an example of the oral cavity image P of the user. The oral cavity image P includes at least a first specific site in the oral cavity. The first specific site includes the central incisor T1, the gum corresponding to the central incisor T1, the lateral incisor T2, the gum corresponding to the lateral incisor T2, the canine T3, and the gum corresponding to the canine T3. The oral cavity image P includes a tooth image and a gum image in the oral cavity. The tooth image includes an image of the central incisor T1, an image of the lateral incisor T2, and an image of the canine T3 in the oral cavity. The gum image includes images of the gums corresponding to the central incisor T1, the lateral incisor T2, and the canine T3. The oral cavity image P includes at least one of the four first specific sites located at upper or lower and left or right positions in the oral cavity of the user.

A preferable range of the tooth image included in the oral cavity image P will be described with reference to Fig. 3. In the determination of the presence or absence of the teeth crowding, it is preferred that the oral cavity image P includes a range from the end portion E1 of the central incisor T1 to the broken line L2. In the determination of the presence or absence of the interdental space, it is preferred that the oral cavity image P includes a range from the end portion E1 of the central incisor T1 to the double-dashed line L3. In the determination of the presence or absence of gingival recession, it is preferred that the oral cavity image P includes a range from the end portion E1 of the central incisor T1 to the solid line L1. When the oral cavity image P includes the image of the central incisor T1 in the range described, the oral cavity image P includes the image of the corresponding gum.

Each oral cavity image P was supervised by an expert who analyzes oral cavities. Figs. 4 to 9 each illustrate an example of a supervised oral cavity image P. An expert who analyzes oral cavities is, for example, a dentist, a dental hygienist, a researcher who studies oral states, or a developer who develops oral care products. The expert who analyzes oral cavities performed a determination of the degree of teeth crowding, a determination of the degree of gingival recession, and a determination of the degree of interdental space for each of the first oral region R1 and the second oral region R2 in the oral cavity image P of the oral cavity. In the determination of the degree of teeth crowding, the expert who analyzes oral cavities performed a determination based on the type of teeth crowding. In the determination of gingival recession, the expert who analyzes oral cavities performed a determination of a plurality of stages from no gingival recession to complete gingival recession. In the determination of the degree of interdental space, the expert who analyzes oral cavities performed an evaluation of a plurality of stages from no interdental space to severe interdental space with respect to each of the upper jaw and the lower jaw.

The state of the oral cavity at the first specific site is correlated with the state of the oral cavity at the second specific site different from the first specific site. In one example, a test conducted on seventy-five women in their 40's to 70's shows that the state of the gingiva in the front tooth and the state of the gingiva in the back tooth are highly correlated. That is, when the gingival recession occurs at the first specific site, the gingival recession also occurs at the second specific site. The same applies to the degree of interdental space and teeth crowding.

The information analysis unit 33 outputs the estimation information E to a predetermined configuration. In the first example, the information analysis unit 33 outputs information to the information output unit 32. In the second example, the information analysis unit 33 outputs information to the information storage unit 34. In the third example, the information analysis unit 33 outputs the estimation information E to both the information output unit 32 and the information storage unit 34.

The estimation information E is associated with the output information O. The output information O will be described. The output information O includes at least one of information regarding a current oral state of the user, information regarding prediction of a future oral state of the user, information regarding an oral care method for the user, and information regarding a health state of the user affected by the oral state of the user. The information regarding the current oral state of the user includes information regarding at least one of presence or absence of teeth crowding, presence or absence of gingival recession, and presence or absence of an interdental space in the entire oral cavity of the user. The information regarding the current oral state of the user may further include at least one of presence or absence of gingival inflammation, presence or absence of insufficient brushing, state of insufficient brushing, presence or absence of bruxism, presence or absence of hyperesthesia, and presence or absence of bad breath.

The information regarding prediction of the future oral cavity of the user includes an estimated image showing an oral state of the user after a predetermined period has elapsed. The information regarding the oral care method for the user includes information regarding an oral care product and a usage method suitable for the oral state of the user. The information regarding the health state of the user affected by the oral state of the user includes, for example, information regarding a health state other than the oral cavity related to the periodontal disease or the oral state.

The correspondence between the estimation information E and the output information O is executed by the information analysis unit 33 with any means. In the first example, it is executed using a correspondence table in which the estimation information E and the output information O are associated with each other in advance by a researcher who studies oral states or a developer who develops oral care products. In the second example, the correspondence between the estimation information E and the output information O is executed using a model of machine learning.

The operation of the oral state evaluation system 10 of the present embodiment will be described. The user inputs the input information I to the acquisition unit 23. The control unit 21 controls the communication unit 24 to output the input information I to the server 30. The server 30 acquires the input information I with the information acquisition unit 31. The information analysis unit 33 calculates the estimation information E from the input information I using the learning model M stored in the information storage unit 34. The information analysis unit 33 calculates the output information O corresponding to the estimation information E. The server 30 outputs the output information O from the information output unit 32 to the interface unit 20. The control unit 21 acquires the output information O from the communication unit 24 and displays the output information O on the display unit 25. This allows the user to recognize the output information O via the display of the display unit 25.

According to the oral state evaluation system 10 of the present embodiment, the following effects can be further obtained.

The information analysis unit 33 analyzes a relationship between the first information and the second information in any combination. Therefore, the analysis using the learning model M executed by the information analysis unit 33 evaluates an oral state that has not been revealed by an analysis of an expert who analyzes oral cavities.

### Modification of Third Embodiment

The description related to the third embodiment exemplifies applicable forms of an oral state evaluation system and the oral care recommendation method according to the present disclosure and is not intended to limit the forms. The present disclosure may take, other than the embodiment, a form in which, for example, modifications of the embodiment shown below and at least two modifications that do not contradict each other are combined.

The learning model M may be configured to output a result of regression analysis. In this case, the learning model M quantifies and outputs at least one of the degree of teeth crowding, the degree of interdental space, and the degree of gingival recession.

At least one of the learned model M1 and the learned model M2 may be a model learned by unsupervised learning or reinforcement learning. At least one of the learning models M11 to M15 and the learning models M21 to M25 may be a model learned by unsupervised learning or reinforcement learning.

The learning model M may include a first learned model M1 that determines whether or not evaluation is possible and a second learned model M2 that performs evaluation without distinguishing the first oral region R1 and the second oral region R2 in the oral cavity image P. The learning model M may be a first learned model M1 that recognizes a set of a tooth and gum in the oral cavity image P and determines whether or not evaluation is possible and a second learned model M2 that performs evaluation. In addition, the learning model M may be a learning model M that recognizes a set of a tooth and gum in the oral region R and executes analysis when a majority of the teeth and gums can be evaluated.

The learning model M may be configured to display the oral cavity image P as the output information O in a pseudo color. In one example, a region used for analysis using the learning model M in the oral cavity image P or a region having interdental space, gingival recession, or teeth crowding are displayed in red. The user can easily recognize the region used for the analysis and the region having a problem with the oral state.

In the second process, any combination of the first information and the second information is selected. The first information and the second information may be different pieces of input information I, and the first information and the second information may be different pieces of estimation information E.

In a case where the output information O includes information regarding the user's oral care product and usage method, it may be configured to include purchase information for purchasing the corresponding oral care product. In one example, the purchase information is information on a store where the corresponding oral care product can be purchased or tried. In another example, the purchase information is site information on a web that sells the corresponding oral care product.

The input information I may be acquired via the loT device. In one example, the input information I is acquired by connecting an loT device to an oral care item used for brushing. In one example, the input information I includes information on the number of times teeth are brushed in a day, and the frequency of use of the oral care item. The loT device may transmit the input information I to the acquisition unit 23, or may transmit the input information I to the server 30.

At least one of the learning model M, the correspondence table, and various types of information may be stored in a place other than the information storage unit 34 of the server 30. In one example, it is stored in a storage unit provided in the interface unit 20. In another example, it is stored in a database configured in an external environment.

The input information I may include first input information I1 including user's current state information and second input information I2 being user's past state information. In a case where the first input information I1 and the second input information I2 include the oral cavity image P of the user, the oral cavity image P included in the second input information I2 is an image of the oral cavity of the user taken at a predetermined time before the first input information I1 is acquired. In a case where the first input information I1 and the second input information I2 include information regarding oral care of the user, the information regarding oral care included in the second input information I2 is information regarding oral care a predetermined time before the first input information I1 is acquired. The predetermined time is, for example, a time of one month or more. In another example, the predetermined time is a time of one year or more. The second input information I2 may be transmitted to the server 30 via the communication unit 24 before the acquisition unit 23 acquires the first input information I1, or may be transmitted to the server 30 via the communication unit 24 simultaneously with the transmission of the first input information I1. In this case, the interface unit 20 further includes a storage unit configured to store at least the second input information I2. The input information I may further include third input information I3 acquired a predetermined time before acquisition of the second input information I2. The information analysis unit 33 calculates the estimation information E using at least one of the pieces of the first input information I1 to the third input information I3. The accuracy is further improved by adding the third input information I3. The interval of the predetermined time may be changed by each piece of input information I. The input information I may further include input information I including fourth input information and so on.

### Example of Applicable Form of Oral State Notification System in Fourth Embodiment

(4-1) An oral state notification system according to the present disclosure includes an information acquisition unit configured to acquire input information from an interface unit; an information analysis unit configured to analyze an oral state of a user based on the input information; and an information output unit configured to output an analysis result obtained from the information analysis unit as output information, where the output information includes future information corresponding to a state of the user after a predetermined time has elapsed.

According to the oral state notification system described above, the output information includes future information corresponding to a state of the user after a predetermined period has elapsed. The user can recognize the state of the user after the predetermined period has elapsed by referring to the future information. Thus, the user can easily recognize whether it is appropriate to continue the oral care that is currently performed or it is appropriate to change the current oral care and perform another method. This encourages to implement an oral caring action.

(4-2) According to an example of the oral state notification system, the future information includes first future information that is information regarding a state of the user in the oral cavity after the predetermined period has elapsed, and the first future information includes at least one of information regarding presence or absence or a degree of interdental space, gingival recession, teeth crowding, the number of natural teeth to be lost, tooth extraction risk, caries, dentin hypersensitivity, bad breath, coloring, tooth wear, tooth loss, and tooth fracture in the oral cavity of the user, information regarding whether or not periodontal disease is present, information regarding whether or not a chewing function is normal, information regarding whether or not occlusion is normal, information regarding whether or not a swallowing function is normal, and information regarding a state of teeth, gums, and oral mucosa in the oral cavity of the user.

According to the oral state notification system described above, the future information includes the first information on the state of the oral cavity of the user. Therefore, a future state in the oral cavity can be easily recognized.

(4-3) According to an example of the oral state notification system, the future information includes second future information different from the first future information, and the second future information includes at least one of information regarding implementation of oral care of the user, information regarding a life risk of the user, and information regarding an oral-related disease of the user.

According to the oral state notification system described above, the user can easily recognize the second future information included in the future information.

(4-4) According to an example of the oral state notification system, the input information includes at least one of an oral cavity image of the user and information regarding oral care of the user.

According to the oral state notification system described above, appropriate future information can be output in correspondence with the input information.

(4-5) According to an example of the oral state notification system, the information regarding the oral care of the user includes at least one of information regarding an oral care item using action of the user, information regarding a lifestyle of the user, information regarding intraoral endocrine of the user, information regarding intraoral bacterial flora of the user, information regarding an attribute of the user, and information obtained by a sensor that detects a state in the oral cavity.

According to the oral state notification system described above, appropriate future information can be output in correspondence with the input information.

(4-6) According to an example of the oral state notification system, the input information includes first input information including current state information of the user and second input information being past state information of the user.

According to the oral state notification system described above, since analysis can be performed by a plurality of pieces of input information, accuracy of the future information improves.

(4-7) According to an example of the oral state notification system, the oral state notification system further includes an information storage unit configured to store a model for analyzing the input information, and the information analysis unit analyzes the input information using the model.

According to the oral state notification system described above, the accuracy of future information is improved by analysis by the model.

(4-8) According to an example of the oral state notification system, the information output unit outputs the output information to at least one of the interface unit and a database that accumulates the output information.

According to the oral state notification system described above, the output information is appropriately used.

### Fourth Embodiment

An oral state notification system 10 according to a fourth embodiment will be described with reference to Figs. 1 to 9. The oral state notification system 10 is a system that analyzes the acquired input information I and outputs the output information O based on the analysis information A obtained from the input information I. The output information O includes future information F corresponding to the state of the user after a predetermined period has elapsed. The oral state notification system 10 may estimate the estimation information E regarding the state of the oral cavity from the analysis information A, and further output the output information O based on the estimation information E. A main element forming the oral state notification system 10 is a server 30. Preferably, the oral state notification system 10 includes an interface unit 20 for exchanging information with the server 30. In one example, the interface unit 20 is a smart device configured to be carried by the user. The smart device includes a tablet terminal or a smartphone. In another example, the interface unit 20 is a personal computer. The personal computer is installed at the user's residence, store, or dental office. A store includes a store that sells oral care items or a store that sells other items. The interface unit 20 and the server 30 are configured to be able to communicate with each other using, for example, the Internet connection N. In another example, the interface unit 20 and the server 30 are integrally configured.

The interface unit 20 includes a control unit 21, a storage unit 22, an acquisition unit 23, a communication unit 24, and a display unit 25. The control unit 21 includes an arithmetic processing unit that executes a predetermined control program. The arithmetic processing unit includes, for example, a CPU, a GPU, or an MPU.

The storage unit 22 stores various control programs executed by the control unit 21 and information used for various control processes. The storage unit 22 includes, for example, a nonvolatile memory and a volatile memory. The nonvolatile memory includes, for example, at least one of a ROM, an EPROM, an EEPROM, and a flash memory. The volatile memory includes, for example, a RAM.

The acquisition unit 23 acquires the input information I from the user. The input information I includes at least one of an oral cavity image P of the user and information regarding oral care of the user. The acquisition unit 23 has any configuration for acquiring the input information I of the user. In the first example, it is a camera capable of acquiring an oral cavity image P of the user's oral cavity. The oral cavity image P includes a still image and a moving image. The oral cavity image P includes a three-dimensional image having depth information or a panoramic image formed by joining a plurality of images. The camera may be a camera mounted on the smart device, or may be a separate independent camera. The independent camera is, for example, a pen type camera in which a camera is provided at a distal end portion suitable for photographing the oral cavity or a camera capable of photographing a range of 360°. The independent camera is configured to be capable of wired or wireless communication with the control unit 21. The oral cavity image P captured by the independent camera is transmitted to the control unit 21 through wired or wireless communication. The acquisition unit 23 of the second example acquires information regarding the oral care of the user. In the second example, the acquisition unit 23 is a user interface configured to allow, for example, the user to input or select a character or the like. The information regarding the oral care of the user includes at least one of information regarding an oral care item using action of the user, information regarding the lifestyle of the user, information regarding the user's intraoral endocrine, information regarding the user's intraoral bacterial flora, information regarding an answer to a predetermined question, and information regarding the attribute of the user. The information regarding an oral care item using action of the user includes at least one of a model number, a frequency of use, the number of times used, and a using time of the user's oral care item. The oral care item includes, for example, at least one of a toothbrush, an interdental cleaning tool, and an oral washing agent. The information regarding the lifestyle of the user includes information regarding the user's meal content and wake-up or sleeping time. The information regarding the intraoral endocrine of the user includes information on the amount of saliva, viscosity, hydrogen ion concentration, amount of antimicrobial component, and amount of component related to tooth remineralization. The information regarding the intraoral bacterial flora of the user includes information regarding the amount and type of main bacteria present in saliva, dental plaque, or mucosa, and information regarding the type and amount of antibodies of the main bacteria. The information regarding the intraoral endocrine of the user and the information regarding the intraoral bacterial flora of the user may be a result of an interview with the user, or may be result information obtained by examining the intraoral endocrine or the like existing in the oral cavity of the user by a predetermined means. The information regarding the answer to the predetermined question includes a question about the state of the user's gums, a preferred brushing method during oral care, whether or not the user feels a sharp pain when eating cold item, whether or not the user is aware of bad breath, the number of current natural teeth, the number of times for brushing teeth in a day, the time taken for brushing teeth each time, the time at which teeth are brushed, the usage frequency of oral care items, and the presence or absence of dentures. The question regarding the state of the user's gums includes, for example, the presence or absence of bleeding from the gums at the time of brushing or eating or drinking. The information regarding the attribute of the user is, for example, the user's own age, sex, height, weight, presence or absence of genetic disease, and medical history. In the third example, the acquisition unit 23 is a sensor that detects a state in the oral cavity. The sensor is, for example, a fluorescent sensor and a temperature sensor. The sensor detects a state in the oral cavity. The fluorescence sensor emits light of a predetermined wavelength, quantifies the amount of light, and measures the distribution and amount of a predetermined object in the oral cavity. The predetermined object is, for example, stain or plaque. The temperature sensor measures a temperature in the oral cavity. The sensor is configured to be capable of wired or wireless communication with the control unit 21. Information obtained by the sensor is transmitted to the control unit 21 through wired or wireless communication. In the present embodiment, the acquisition unit 23 may be configured by combining two or more of the first to third examples. The sensor may be further configured to be able to measure at least one of chewing force, biting force, amount of bleeding from gums, bad breath, strength of brush pressure at the time of brushing, or movement of toothbrush at the time of brushing.

The input information I includes first input information I1 including user's current state information and second input information I2 being user's past state information. In a case where the first input information I1 and the second input information I2 include the oral cavity image P of the user, the oral cavity image P included in the second input information I2 is an image of the oral cavity of the user taken at a predetermined time before the first input information I1 is acquired. In a case where the first input information I1 and the second input information I2 include information regarding oral care of the user, the information regarding oral care included in the second input information I2 is information regarding oral care a predetermined time before the first input information I1 is acquired. The predetermined time is, for example, a time of one month or more. In another example, the predetermined time is a time of one year or more. The second input information I2 may be transmitted to the server 30 via the communication unit 24 before the acquisition unit 23 acquires the first input information I1, or may be transmitted to the server 30 via the communication unit 24 simultaneously with the transmission of the first input information I1. In this case, the interface unit 20 further includes a storage unit configured to store at least the second input information I2.

The communication unit 24 is configured to be able to communicate with the outside of the interface unit 20 based on the control of the control unit 21. The communication unit 24 is configured to be able to communicate via the Internet connection N. The communication unit 24 may be configured to be able to communicate with the server 30 of the interface unit 20 through wired communication or wireless communication. The communication unit 24 transmits, for example, the input information I of the user acquired by the acquisition unit 23 based on the control of the control unit 21, and receives the output information O from the server 30.

The display unit 25 displays various types of information based on the control of the control unit 21. The various types of information are, for example, information regarding the input information I of the user and information regarding the output information O from the server 30. In one example, the display unit 25 includes a display. The display of the display unit 25 may include a touch panel. In a case where a part of the display unit 25 is formed of a touch panel, that part may also function as a user interface of the acquisition unit 23.

The user communicates with the server 30 by, for example, inputting a predetermined URL to the interface unit 20 or reading a QR code (registered trademark) by the interface unit 20. The user may start communication with the server 30 by selecting an icon displayed on the display unit 25.

The server 30 includes an information acquisition unit 31, an information output unit 32, an information analysis unit 33, and an information storage unit 34. The information acquisition unit 31 is configured to be able to acquire information. In one example, the information acquisition unit 31 acquires information from the communication unit 24 of the interface unit 20. The information output unit 32 is configured to be able to output information. In one example, the information output unit 32 outputs information to the communication unit 24 of the interface unit 20. In another example, the information output unit 32 outputs information to the database.

The information analysis unit 33 executes various analyses and controls. The information analysis unit 33 includes an arithmetic processing unit that executes a predetermined control program. The arithmetic processing unit includes, for example, a CPU or an MPU. The information analysis unit 33 is configured to be able to analyze the input information I from the user. In the first example, the information analysis unit 33 analyzes the input information I using the learning model M by machine learning. The input information I in this case includes the oral cavity image P of the user. In one example, the learning model M is a supervised learning model, which is one of the models of machine learning. In the second example, the information analysis unit 33 analyzes the input information I with reference to a correspondence table stored in the information storage unit 34. The input information I in this case includes information regarding oral care of the user instead of or in addition to the oral cavity image P. The correspondence table is a table in which the input information I and the analysis information A are associated with each other. In the correspondence table, the input information I and the analysis information A may be associated with the estimation information E. In a case where the input information I is further included in addition to the oral cavity image P, in one example, the learning model M further includes another learning model that analyzes the input information I other than the oral cavity image P. Another learning model is configured to be able to output, for example, a parameter for correcting the analysis information A of the oral cavity image P. In another example, the learning model M is configured as a model capable of executing multimodal learning for performing learning by combining both the oral cavity image P and the input information I not including the oral cavity image P. In a case where the input information I includes the first input information I1 and the second input information I2, analysis is performed for each piece of the input information I, and a plurality of pieces of corresponding analysis information A and a plurality of pieces of corresponding estimation information E are calculated. When the input information I includes the first input information I1 and the second input information I2, one piece of corresponding analysis information A and a plurality of pieces of corresponding estimation information E may be calculated by a plurality of pieces of input information I.

The information storage unit 34 stores at least one of the learning model M, the correspondence table, and various types of information. The information analysis unit 33 refers to the learning model M, the correspondence table, and various types of information stored in the information storage unit 34 as necessary. The information storage unit 34 includes, for example, a nonvolatile memory and a volatile memory. The nonvolatile memory includes, for example, at least one of a ROM, an EPROM, an EEPROM, and a flash memory. The volatile memory includes, for example, a RAM.

The analysis of the input information I including the oral cavity image P using the learning model M executed by the information analysis unit 33 will be described. The step of analysis executed by the information analysis unit 33 includes a number of processes. The processes include a first process of detecting an oral region R from the oral cavity image P of the user, a second process of calculating the analysis information A by analyzing the oral region R included in the oral cavity image P, a third process of estimating the estimation information E from the analysis information A, and a fourth process of calculating the output information O corresponding to the analysis information A and the estimation information E. The acquisition of the estimation information E in the third process has an effect of improving the accuracy of the output information O.

The first process is executed by the information analysis unit 33 with any means. In one example, the oral region R is detected from the oral cavity image P of the oral cavity of the user using a learned model suitable for detecting the face. The oral region R includes a first oral region R1 including the teeth of the user's upper jaw and a second oral region R2 including the teeth of the user's lower jaw. The learned model for detecting the face is introduced into the information analysis unit 33 by, for example, the API.

In the first process, in a case where the oral region R cannot be acquired from the oral cavity image P, the information analysis unit 33 outputs a content indicating the result to the display unit 25 of the interface unit 20 via the information output unit 32. In one example, when the oral region R cannot be recognized because the oral region R is not included in the oral cavity image P, the oral cavity image P is blurry, or the brightness of the oral cavity image P is not appropriate, the display unit 25 of the interface unit 20 outputs information requesting the user to again input the input information I including the oral cavity image P. In a case where one of the first oral region R1 and the second oral region R2 can be acquired and the other cannot be acquired, the information analysis unit 33 may determine that the first process has been completed, or may output, to the interface unit 20, information requesting the user to again input the input information I.

In the second process, the information analysis unit 33 analyzes the oral cavity image P. The information analysis unit 33 analyzes the oral cavity image P using the learning model M stored in the information storage unit 34. The analysis information A, which is the analysis result, includes at least one of information regarding presence or absence or a degree of interdental space, gingival recession, teeth crowding, gingival inflammation, insufficient brushing, bruxism, caries, hyperesthesia, bad breath, and coloring at the first specific site in the oral region R, and information regarding a state of teeth, gums, and oral mucosa corresponding to the first specific site. The information regarding the state of the teeth, gums, and oral mucosa includes at least one of, for example, the presence or absence or degree of tooth wear, tooth loss, and tooth fracture, color, dryness, and texture. The texture includes, for example, a plurality of textures including a hard texture that is felt to be firm when touched with a finger and a soft texture that is felt to be tender when touched with a finger. In a case where the results differ between the analysis information A in the first oral region R1 and the analysis information A in the second oral region R2, one of the two types of the analysis information A may be set in advance to be used. Alternatively, the two types of analysis information A may be quantified and an average thereof may be used. The two types of analysis information A may be quantified and the analysis information A having a larger absolute value may be used.

In the third process, the information analysis unit 33 estimates the estimation information E from the input information I and the analysis information A. The estimation information E includes information on at least one of the presence or absence, the degree, and the probability of a predetermined state. The estimation information E includes at least one of information regarding presence or absence or a degree of interdental space, gingival recession, teeth crowding, gingival inflammation, insufficient brushing, bruxism, caries, hyperesthesia, bad breath, and coloring at the second specific site different from the first specific site, or information regarding a state of teeth, gums, and oral mucosa corresponding to the second specific site. The second specific site includes at least one of a premolar and a molar in the oral cavity. The estimation information E includes at least one of information on a premolar and information regarding a molar in the oral cavity. The estimation information E may include at least one of information regarding whether or not periodontal disease is present in the oral cavity of the user, information regarding whether or not chewing function is normal, information regarding whether or not occlusion is normal, and information regarding whether or not swallowing function is normal, The information on whether or not periodontal disease is present is information on at least one of the size, depth, and number of so-called periodontal pockets that are formed between the gingiva and the tooth. The information on whether or not the chewing function is normal is, for example, information on chewing force, presence or absence of unbalanced chewing, and chewing sound. The information on whether or not occlusion is normal is, for example, information on the biting force and the fitting of the tooth of the upper jaw with the tooth of the lower jaw. The information on whether or not the swallowing function is normal is, for example, information on presence or absence of swallowing difficulty.

The learning model M for analyzing the oral region R will be described. The learning model M includes a plurality of learned models. The learning model M includes a first learned model M1 that determines whether or not the oral region R can be evaluated and a second learned model M2 that actually evaluates the oral region R.

The first learned model M1 includes at least one of a learning model M11 that determines whether or not the oral region R of the oral cavity image P has an analyzable image quality, a learning model M12 that determines whether or not the teeth crowding evaluation is possible, a learning model M13 that determines whether or not the interdental space evaluation of the teeth of the upper jaw is possible, a learning model M14 that determines whether or not the interdental space evaluation of the teeth of the lower jaw is possible, and a learning model M15 that determines whether or not the gingival recession evaluation is possible.

The second learned model M2 includes at least one of a learning model M21 that determines presence or absence of teeth crowding in the tooth of the upper jaw, a learning model M22 that determines presence or absence of teeth crowding in the tooth of the lower jaw, a learning model M23 that determines presence or absence of an interdental space in the tooth of the upper jaw, a learning model M24 that determines presence or absence of an interdental space in the tooth of the lower jaw, and a learning model M25 that determines presence or absence of interdental recession.

The creation of the learning model M will be described. The learning model M was created based on approximately 10,000 oral cavity images P. The developer classified the oral cavity image P into a learning image, a verification image, and a test image. The learning image is supervised data at the time of creating the learning model M. The verification image is an image for correcting the operation of the learning model M based on the learning image. The test image is an image for finally confirming the operation of the learning model M. The test image is used, for example, to determine whether or not the learning model M is causing overfitting. The learning image accounts for about 56% of the whole. The verification image accounts for about 24% of the whole. The test image accounts for 20% of the whole. In the present embodiment, the learning model M outputs a result of class classification. The learning model M outputs at least one of information regarding presence or absence or a degree of interdental space, gingival recession, teeth crowding, gingival inflammation, insufficient brushing, bruxism, caries, hyperesthesia, bad breath, and coloring, information regarding a state of gums and oral mucosa corresponding to the second specific site, information regarding whether or not periodontal disease is present in the oral cavity of the user, information regarding whether or not chewing function is normal, information regarding whether or not occlusion is normal, and information regarding whether or not swallowing function is normal.

Fig. 2 illustrates an example of the oral cavity image P of the user. The oral cavity image P includes at least a first specific site in the oral cavity. The first specific site includes the central incisor T1, the gum corresponding to the central incisor T1, the lateral incisor T2, the gum corresponding to the lateral incisor T2, the canine T3, and the gum corresponding to the canine T3. The oral cavity image P includes a tooth image and a gum image in the oral cavity. The tooth image includes an image of the central incisor T1, an image of the lateral incisor T2, and an image of the canine T3 in the oral cavity. The gum image includes images of the gums corresponding to the central incisor T1, the lateral incisor T2, and the canine T3. The oral cavity image P includes at least one of the four first specific sites located at upper or lower and left or right positions in the oral cavity of the user.

A preferable range of the tooth image included in the oral cavity image P will be described with reference to Fig. 3. In the determination of the presence or absence of the teeth crowding, it is preferred that the oral cavity image P includes a range from the end portion E1 of the central incisor T1 to the broken line L2. In the determination of the presence or absence of the interdental space, it is preferred that the oral cavity image P includes a range from the end portion E1 of the central incisor T1 to the double-dashed line L3. In the determination of the presence or absence of gingival recession, it is preferred that the oral cavity image P includes a range from the end portion E1 of the central incisor T1 to the solid line L1. When the oral cavity image P includes the image of the central incisor T1 in each range described above, the oral cavity image P includes the image of the corresponding gum.

Each oral cavity image P was supervised by an expert who analyzes the oral cavities. Figs. 4 to 9 each illustrate an example of a supervised oral cavity image P. An expert who analyzes oral cavities is, for example, a dentist, a dental hygienist, a researcher who studies oral states, or a developer who develops oral care items. The expert who analyzes oral cavities performed a determination of the degree of teeth crowding, a determination of the degree of gingival recession, and a determination of the degree of interdental space for each of the first oral region R1 and the second oral region R2 in the oral cavity image P of the oral cavity. In the determination of the degree of teeth crowding, the expert who analyzes oral cavities performed a determination based on the type of teeth crowding. In the determination of gingival recession, the expert who analyzes oral cavities performed a determination of a plurality of stages from no gingival recession to complete gingival recession. In the determination of interdental space, the expert who analyzes oral cavities performed an evaluation of a plurality of stages from no interdental space to severe interdental space with respect to each of the upper jaw and the lower jaw.

The state of the oral cavity at the first specific site is correlated with the state of the oral cavity at the second specific site different from the first specific site. In one example, a test conducted on seventy-five women in their 40's to 70's shows that the state of the gingiva in the front tooth and the state of the gingiva in the back tooth are highly correlated. That is, when the gingival recession occurs at the first specific site, the gingival recession also occurs at the second specific site. The same applies to the degree of interdental space and teeth crowding.

The information analysis unit 33 calculates the future information F based on at least one of the input information I, the analysis information A, and the estimation information E. The future information F includes first future information F1 that is information regarding the state of the user's oral cavity after a predetermined period has elapsed. The predetermined period is set in any manner. In one example, the predetermined period is a period of one year or more. The first future information F1 includes information regarding at least one of the presence or absence, the degree, and the probability of the predetermined state in the oral cavity. The first future information F1 includes at least one of information regarding presence or absence or a degree of interdental space, gingival recession, teeth crowding, the number of natural teeth to be lost, tooth extraction risk, caries, dentin hypersensitivity, bad breath, and coloring in the oral cavity of the user, information regarding whether or not periodontal disease is present, information regarding whether or not a chewing function is normal, information regarding whether or not occlusion is normal, information regarding whether or not a swallowing function is normal, and information regarding a state of teeth, gums, and oral mucosa in the oral cavity of the user. The tooth extraction risk is, for example, a probability that the tooth needs to be extracted due to caries or gingival recession.

A method of calculating the first future information F1 executed by the information analysis unit 33 will be described by way of example. The information analysis unit 33 may calculate the first future information F1 by any one of the calculation methods exemplified below, or may calculate the first future information F1 by two or more calculation methods.

In the first example, the information analysis unit 33 calculates the future information F in accordance with the correspondence between at least one of the input information I, the analysis information A, and the estimation information E and the correspondence table. The correspondence table is stored in, for example, the information storage unit 34. The correspondence table may be acquired by the information acquisition unit 31 from an external environment. In one example, the input information I includes the current number of natural teeth as the first input information I1, and includes the past number of natural teeth as the second input information I2. In the correspondence table, the vertical axis represents the number of natural teeth, and the horizontal axis represents age. The following table is an example of the correspondence table.

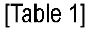

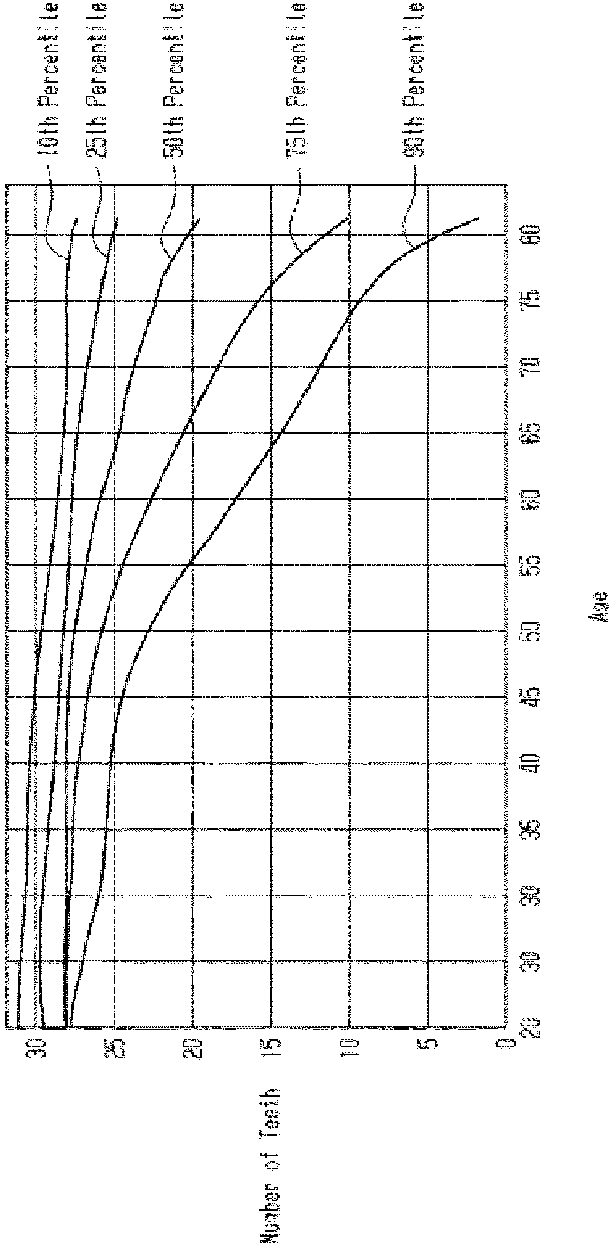

The information analysis unit 33 calculates the first future information F1 by referring to the correspondence table. The first future information F1 includes information on the number of natural teeth at a predetermined age, that is, the number of natural teeth lost at a predetermined age.

In the second example, the information analysis unit 33 calculates at least one of the input information I, the analysis information A, and the estimation information E using a statistical modeling method, and calculates the first future information F1. Specifically, logistic regression analysis and multiple regression analysis are executed. Preferably, the input information I includes both the first input information I1 and the second input information I2, and a plurality of pieces of analysis information A and a plurality of pieces of estimation information E calculated from each piece of the input information I are used.

In the third example, the information analysis unit 33 calculates at least one of the input information I, the analysis information A, and the estimation information E using a prediction model method of machine learning, and calculates the first future information F. Any prediction model method of machine learning may be used. For example, the prediction model method is a supervised learning model. Examples include a regression analysis model, a decision tree model, a neural network model including deep learning, a Naive Bayes method, an autoregressive method, a state space model, a K-neighborhood model, a support vector machine, and ensemble learning. The prediction model method may be an unsupervised learning model. Preferably, the input information I includes both the first input information I1 and the second input information I2, and a plurality of pieces of analysis information A and a plurality of pieces of estimation information E calculated from each piece of the input information I are used.

The future information F further includes second future information F2 different from the first future information F1. The second future information F2 is information other than the state of the oral cavity of the user. The second future information F2 includes at least one of information regarding implementation of oral care of the user, information regarding a life risk of the user, and information regarding an oral-related disease of the user.

The information regarding implementation of oral care of the user includes, for example, information regarding temporal and financial costs required for implementation of care. The implementation of the care is for example, the treatment of periodontal disease and implants. The information regarding implementation of oral care of the user may include information regarding tools necessary for implementation of care. The information regarding the life risk of the user includes information that becomes a risk when the user lives normally after a predetermined period has elapsed. The information regarding the life risk of the user includes, for example, information regarding ease of eating solid objects, ease of vocalization, and ease of making facial expressions. The information regarding the oral-related disease of the user includes information regarding a disease assumed to be caused by the state of the oral cavity. The information regarding the oral-related disease of the user includes, for example, information regarding diabetes, dementia, and aspiration pneumonia.

The information analysis unit 33 calculates the second future information F2 based on at least one of the first future information F1 and the additional information. The additional information is auxiliary information for calculating the second future information F2. In the first example, the additional information is a table in which the temporal and financial costs corresponding to the degree of periodontal disease can be calculated. In the second example, the additional information is a table showing the relationship between the chewing force and the ease of eating, or a table associating the degree of teeth crowding with the ease of vocalization. In the third example, the additional information is academic information indicating a relationship between periodontal disease and other states and diseases in the oral cavity. The additional information may be stored in the information storage unit 34 or may be saved in a database configured in an external environment, and the information analysis unit 33 may be configured to appropriately acquire the additional information.

The information analysis unit 33 outputs the analysis information A, the estimation information E, and the future information F to a predetermined configuration. In the first example, the information analysis unit 33 outputs information to the information output unit 32. In the second example, the information analysis unit 33 outputs information to the information storage unit 34. In the third example, the information analysis unit 33 outputs information to both the information output unit 32 and the information storage unit 34.

The information output unit 32 outputs the output information O including the future information F to at least one of the interface unit 20 and a database that accumulates the output information O. In a case where the output information O is output to the interface unit 20, the display unit 25 of the interface unit 20 preferably displays at least one of the output information O as a character or an image.

The database is provided, for example, in an internal or external environment of the oral state notification system 10. The output information O obtained by the oral state notification system 10 is accumulated in the database. Preferably, the databases are classified by group. In one example, the group is set based on information regarding the attribute of the user. The group may be set based on the place of residence or occupation of the user, or may be set based on use of a predetermined treatment facility.

An operation of oral state notification system 10 of the present embodiment will be described. The user inputs the input information I to the acquisition unit 23. The control unit 21 controls the communication unit 24 to output the input information I to the server 30. The server 30 acquires the input information I with the information acquisition unit 31. The information analysis unit 33 analyzes the input information I and calculates the analysis information A. The information analysis unit 33 estimates the estimation information E from the analysis information A. The information analysis unit 33 calculates the output information O including the future information F from the input information I, the analysis information A, and the estimation information E. The server 30 outputs the output information O from the information output unit 32 to the interface unit 20. The control unit 21 acquires the output information O from the communication unit 24 and displays the output information O on the display unit 25. This allows the user to recognize the future information F included in the output information O via the display of the display unit 25.

### Modification of Fourth Embodiment

The description related to the fourth embodiment exemplifies applicable forms of an oral state notification system according to the present disclosure and is not intended to limit the forms. The present disclosure may take, other than the embodiment, a form in which, for example, modifications of the embodiment shown below and at least two modifications that do not contradict each other are combined.

The learning model M may be configured to output a result of regression analysis. In this case, the learning model M quantifies and outputs at least one of the degree of teeth crowding, the degree of interdental space, and the degree of gingival recession.

At least one of the learned model M1 and the learned model M2 may be a model learned by unsupervised learning or reinforcement learning. At least one of the learning models M11 to M15 and the learning models M21 to M25 may be a model learned by unsupervised learning or reinforcement learning.

The learning model M may include a first learned model M1 that determines whether or not evaluation is possible and a second learned model M2 that performs evaluation without distinguishing the first oral region R1 and the second oral region R2 in the oral cavity image P. The learning model M may include a first learned model M1 that recognizes a set of a tooth and gum in the oral cavity image P and determines whether or not evaluation is possible and a second learned model M2 that performs evaluation.

The learning model M may be configured to display the oral cavity image P as the output information O in a pseudo color. In one example, a region used for analysis using the learning model M in the oral cavity image P or a region having interdental space, gingival recession, or teeth crowding are displayed in red. The user can easily recognize the region used for the analysis and the region having a problem with the oral state.

When the input information I includes the oral cavity image P, the future information F may include an estimated image. The estimated image is an oral cavity image of the user calculated based on the input information I after a predetermined time has elapsed. In one example, in a case where the amount of stains or plaques in the oral cavity image P of the first input information I1 is greater than the amount of stains or plaques in the oral cavity image P of the second input information I2, the estimated image is displayed such that the gingival recession occurs more and stains or plaques are distributed in a wider range than in the oral cavity image P. The estimated image is, for example, displayed on the display unit 25. This allows the user to easily recognize the future oral state, thereby encouraging to use the oral care item.

The oral cavity image P may be a moving image in which movements in and around the oral cavity are captured, or a moving image including movements when the user uses the oral care item. The oral cavity image P may be an image including both the first specific site and the second specific site in the oral cavity, or may be an image including only the second specific site.

The output information O may further include information regarding the current oral state of the user in addition to the future information F. The information regarding the current oral state of the user includes at least one of information regarding presence or absence or a degree of interdental space, gingival recession, teeth crowding, gingival inflammation, insufficient brushing, bruxism, caries, hyperesthesia, bad breath, and coloring, information regarding whether or not periodontal disease is present in the oral cavity of the user, information regarding whether or not chewing function is normal, information regarding whether or not occlusion is normal, information regarding whether or not swallowing function is normal, and information regarding a state of teeth, gums, and oral mucosa corresponding to the second specific site.

The output information O may include recommendation information RI regarding oral care of the user. The recommendation information RI includes at least oral care item recommendation information RI1 for recommending an oral care item in accordance with the oral state of the user. The recommendation information RI may further include information RI2 regarding usage method of the oral care item. The information RI2 regarding the usage method of the oral care item may be a usage method of the oral care item included in the oral care item recommendation information RI1, or may be a general usage method of an oral care item that is not included in the oral care item recommendation information RI1. In a case where the output information O includes information regarding the user's oral care product and usage method, it may be configured to include purchase information for purchasing the corresponding oral care product. In one example, the purchase information is information on a store where the corresponding oral care product can be purchased or tried. In another example, the purchase information is site information on a web that sells the corresponding oral care product.

At least one of the learning model M, the correspondence table, and various types of information may be stored in a place other than the information storage unit 34 of the server 30. In one example, it is stored in a storage unit provided in the interface unit 20. In another example, it is stored in a database configured in an external environment.

The input information I may further include third input information I3 acquired a predetermined time before acquisition of the second input information I2. The information analysis unit 33 calculates the analysis information A and the estimation information E using the third input information I3. The accuracy of the first future information F1 is further improved by adding the third input information I3. The interval of the predetermined time may be changed by each piece of input information I. The input information I may further include input information I including fourth input information and so on.

The oral state notification system 10 may be configured to further analyze the output information O accumulated in the database. In one example, the information analysis unit 33 further analyzes the output information O accumulated in the database. The future information F in the group to which the user belongs is calculated by analyzing the accumulated output information O. It may be configured to compare future information F of a plurality of groups including a group to which the user does not belong and output a comparison result.

The input information I may be acquired via the loT device. In one example, the input information I is acquired by connecting an loT device to an oral care item used for brushing. In one example, the input information I includes information on the number of times teeth are brushed in a day, and the frequency of use of the oral care item. The loT device may transmit the input information I to the acquisition unit 23, or may transmit the input information I to the server 30.

In the first to fourth embodiments, the interface unit 20 and the server 30 are not limited to those that perform software processing for all of the processes executed by the interface unit 20 and the server 30. For example, the interface unit 20 and the server 30 may include a dedicated hardware circuit (e.g., an application specific integrated circuit (ASIC)) that performs hardware processing for at least some of the processes. That is, the interface unit 20 and the server 30 can be configured as circuitry including 1) one or more processors that operates in accordance with a computer program (software), 2) one or more dedicated hardware circuits that execute at least some of the various processes, or 3) a combination thereof. The processor includes a CPU and a memory such as a RAM and a ROM, and the memory stores a program code or a command configured to cause the CPU to execute a process. The memory, or a computer readable medium, includes any available medium that can be accessed by a general purpose or dedicated computer.

### REFERENCE SIGNS LIST

10: oral state evaluation system, oral care recommendation system, and oral state notification system
20: interface unit
30: server
31: information acquisition unit
32: information output unit
33: information analysis unit
34: information storage unit
A: analysis information
E: estimation information
I: input information
O: output information
RI: recommendation information

## Claims

1. An oral state evaluation system comprising:
an information acquisition unit configured to acquire an oral cavity image including at least a first specific site in an oral cavity as input information from an interface unit; and
an information analysis unit configured to analyze a state of the first specific site based on the input information, wherein
the information analysis unit estimates estimation information regarding a state of a second specific site in the oral cavity different from the first specific site from analysis information of the state of the first specific site.

2. The oral state evaluation system according to claim 1, wherein the oral cavity image includes a tooth image and a gum image in the oral cavity.

3. The oral state evaluation system according to claim 2, wherein the tooth image includes a central incisor image, a lateral incisor image, and a canine image in the oral cavity.

4. The oral state evaluation system according to any one of claims 1 to 3,
wherein
the second specific site includes at least one of a premolar and a molar in the oral cavity, and
the estimation information includes at least one of information regarding the premolar and information regarding the molar in the oral cavity.

5. The oral state evaluation system according to any one of claims 1 to 4,
wherein
the estimation information includes at least one of information regarding presence or absence or a degree of interdental space, gingival recession, teeth crowding, gingival inflammation, insufficient brushing, bruxism, caries, hyperesthesia, bad breath, and coloring at the second specific site, information regarding whether periodontal disease is present, information regarding whether chewing function is normal, information regarding whether occlusion is normal, information regarding whether swallowing function is normal, and information regarding a state of teeth, gums, and oral mucosa corresponding to the second specific site.

6. The oral state evaluation system according to any one of claims 1 to 5, further comprising
an information storage unit configured to store a learning model in which the oral cavity image is learned in advance to evaluate a state in the oral cavity, wherein
the information analysis unit analyzes the input information with the learning model.

7. The oral state evaluation system according to any one of claims 1 to 6,
wherein the input information further includes at least one of information regarding a lifestyle of a user, information regarding an intraoral endocrine of the user, information regarding an intraoral bacterial flora of the user, information regarding an attribute of the user, and information obtained by a sensor that detects a state in the oral cavity.

8. The oral state evaluation system according to any one of claims 1 to 7, further comprising
an information output unit configured to output information corresponding to the analysis information and the estimation information as output information, wherein
the information output unit outputs the output information to at least the interface unit.

9. The oral state evaluation system according to claim 8, wherein the output information includes at least one of information regarding a current oral state of a user, information regarding prediction of a future oral state of the user, information regarding an oral care method for the user, and information regarding a health state of the user affected by the oral state of the user.

10. An oral care recommendation system comprising:
an information acquisition unit configured to acquire input information from an interface unit;
an information analysis unit configured to analyze an oral state of a user based on the input information; and
an information output unit configured to output an analysis result obtained from the information analysis unit as output information, wherein
the output information includes recommendation information regarding oral care of the user.

11. The oral care recommendation system according to claim 10, wherein
the recommendation information includes oral care item recommendation information for recommending an oral care item in accordance with the oral state of the user.

12. The oral care recommendation system according to claim 11, wherein the oral care item includes at least one of a toothbrush, an interdental cleaning tool, and an oral washing agent.

13. The oral care recommendation system according to claim 11 or 12, wherein the recommendation information further includes information regarding a usage method of the oral care item.

14. The oral care recommendation system according to claim 13, wherein
the input information includes an image including a first specific site in an oral cavity of the user, and
the information regarding a usage method of the oral care item includes a usage method of the oral care item at least at the first specific site.

15. The oral care recommendation system according to any one of claims 10 to 14, wherein
the input information includes information regarding the oral care of the user, and
the output information further includes answer information corresponding to the information regarding the oral care of the user.

16. The oral care recommendation system according to claim 15, wherein
the information regarding the oral care of the user includes at least one of information regarding an oral care item using action of the user, information regarding a lifestyle of the user, information regarding an intraoral endocrine of the user, information regarding an intraoral bacterial flora of the user, information regarding an attribute of the user, and information obtained by a sensor that detects a state in the oral cavity.

17. The oral care recommendation system according to any one of claims 10 to 16, wherein
the information output unit outputs the output information to the interface unit, and
the interface unit includes a display unit configured to display the output information.

18. An oral state evaluation system comprising:
an information acquisition unit configured to acquire an oral cavity image including at least a first specific site in an oral cavity as input information from an interface unit; and
an information analysis unit configured to analyze the input information by a learning model that has learned the oral cavity image in advance to evaluate the state in the oral cavity, wherein
the information analysis unit estimates estimation information regarding a state at a second specific site in the oral cavity different from the first specific site.

19. The oral state evaluation system according to claim 18, wherein the oral cavity image includes a tooth image and a gum image in the oral cavity.

20. The oral state evaluation system according to claim 19, wherein the tooth image includes a central incisor image, a lateral incisor image, and a canine image in the oral cavity.

21. The oral state evaluation system according to any one of claims 18 to 20, wherein
the second specific site includes at least one of a premolar and a molar in the oral cavity, and
the estimation information includes at least one of information regarding the premolar and information regarding the molar in the oral cavity.

22. The oral state evaluation system according to any one of claims 18 to 21,
wherein
the estimation information includes at least one of information regarding presence or absence or a degree of interdental space, gingival recession, teeth crowding, gingival inflammation, insufficient brushing, bruxism, caries, hyperesthesia, bad breath, and coloring at the second specific site, information regarding whether periodontal disease is present, information regarding whether chewing function is normal, information regarding whether occlusion is normal, information regarding whether swallowing function is normal, and information regarding a state of teeth, gums, and oral mucosa corresponding to the second specific site.

23. The oral state evaluation system according to any one of claims 18 to 22, further comprising an information storage unit configured to store the learning model.

24. The oral state evaluation system according to any one of claims 18 to 23,
wherein
the input information further includes at least one of information regarding a lifestyle of a user, information regarding an intraoral endocrine of the user, information regarding an intraoral bacterial flora of the user, information regarding an attribute of the user, and information obtained by a sensor that detects a state in the oral cavity.

25. The oral state evaluation system according to any one of claims 18 to 24, further comprising
an information output unit configured to output information corresponding to the estimation information as output information, wherein
the information output unit outputs the output information to at least the interface unit.

26. The oral state evaluation system according to claim 25, wherein
the output information includes at least one of information regarding a current oral state of a user, information regarding prediction of a future oral state of the user, information regarding an oral care method for the user, and information regarding a health state of the user affected by the oral state of the user.

27. An oral state notification system comprising:
an information acquisition unit configured to acquire input information from an interface unit;
an information analysis unit configured to analyze an oral state of a user based on the input information; and
an information output unit configured to output an analysis result obtained from the information analysis unit as output information, wherein
the output information includes future information corresponding to a state of the user after a predetermined time has elapsed.

28. The oral state notification system according to claim 27, wherein
the future information includes first future information that is information regarding a state of the user in the oral cavity after the predetermined period has elapsed, and
the first future information includes at least one of information regarding presence or absence or a degree of interdental space, gingival recession, teeth crowding, the number of natural teeth to be lost, tooth extraction risk, caries, dentin hypersensitivity, bad breath, and coloring in the oral cavity of the user, information regarding whether periodontal disease is present, information regarding whether chewing function is normal, information regarding whether occlusion is normal, information regarding whether swallowing function is normal, and information regarding a state of teeth, gums, and oral mucosa in the oral cavity of the user.

29. The oral state notification system according to claim 27 or 28, wherein
the future information includes second future information different from the first future information, and
the second future information includes at least one of information regarding implementation of oral care of the user, information regarding a life risk of the user, and information regarding an oral-related disease of the user.

30. The oral state notification system according to any one of claims 27 to 29,
wherein the input information includes at least one of an oral cavity image of the user and information regarding oral care of the user.

31. The oral state notification system according to claim 30, wherein the information regarding the oral care of the user includes at least one of information regarding an oral care item using action of the user, information regarding a lifestyle of the user, information regarding an intraoral endocrine of the user, information regarding an intraoral bacterial flora of the user, information regarding an attribute of the user, and information obtained by a sensor that detects a state in the oral cavity.

32. The oral state notification system according to any one of claims 27 to 31, wherein the input information includes first input information including current state information of the user and second input information being past state information of the user.

33. The oral state notification system according to any one of claims 27 to 32, further comprising
an information storage unit configured to store a model for analyzing the input information, wherein
the information analysis unit analyzes the input information using the model.

34. The oral state notification system according to any one of claims 27 to 33, wherein the information output unit outputs the output information to at least one of the interface unit and a database that accumulates the output information.
